(19) Europäisches Patentamt
European Patent Office
Office européen des brevets

(11) EP 4 491 617 A1

(12) EUROPEAN PATENT APPLICATION
published in accordance with Art. 153(4) EPC

(43) Date of publication:
**15.01.2025 Bulletin 2025/03**

(21) Application number: **23765951.1**

(22) Date of filing: **06.03.2023**

(51) International Patent Classification (IPC):
***C07D 213/81*** (2006.01) ***C07D 401/12*** (2006.01)
***C07D 401/02*** (2006.01) ***A61K 31/443*** (2006.01)
***A61K 31/4433*** (2006.01) ***A61K 31/4439*** (2006.01)
***A61K 31/444*** (2006.01) ***A61P 35/00*** (2006.01)
***A61P 37/02*** (2006.01)

(52) Cooperative Patent Classification (CPC):
**A61K 31/443; A61K 31/4433; A61K 31/4439; A61K 31/444; A61P 35/00; A61P 37/02; C07D 213/81; C07D 401/02; C07D 401/12**

(86) International application number:
**PCT/CN2023/079912**

(87) International publication number:
**WO 2023/169373 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.03.2022 CN 202210216572**

(71) Applicant: **Abbisko Therapeutics Co., Ltd.**
**Shanghai 201203 (CN)**

(72) Inventors:
- **YANG, Fei**
  **Shanghai 201203 (CN)**
- **ZHANG, Yongxian**
  **Shanghai 201203 (CN)**
- **YU, Hongping**
  **Shanghai 201203 (CN)**
- **CHEN, Zhui**
  **Shanghai 201203 (CN)**
- **XU, Yaochang**
  **Shanghai 201203 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **HOLOSYMMETRIC BIPHENYL DERIVATIVE, AND PREPARATION METHOD THEREFOR AND USE THEREOF**

(57) The present invention relates to a holosymmetric biphenyl derivative, and a preparation method therefor and the use thereof. Particularly, the present invention relates to a holosymmetric biphenyl derivative having the structure of formula (I), and a preparation method therefor and a pharmaceutical composition containing same. The series of compounds of the present invention can be widely used for the preparation of a drug for preventing and/or treating PD-1/PD-L1 signal pathway-mediated cancers or tumors, immune-related diseases and disorders, communicable diseases, infectious diseases or metabolic diseases, and are expected to be developed into a new generation of PD-1/PD-L1 inhibitors.

EP 4 491 617 A1

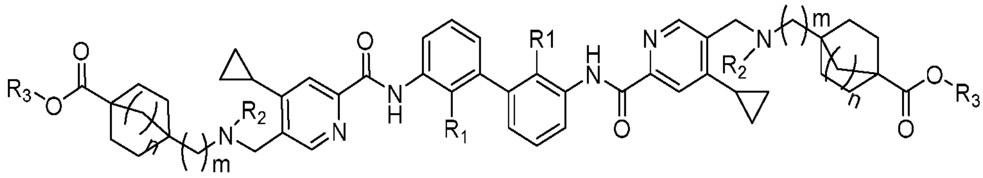

(I)

## Description

### TECHNICAL FIELD

The present invention belongs to the field of pharmaceutical synthesis, and particularly relates to a holosymmetric biphenyl derivative and a preparation method therefor and use thereof.

### BACKGROUND

The immune system plays a very important role in controlling and eliminating diseases, such as cancer. However, tumor cells are often able to develop a strategy for escaping or suppressing the monitoring of the immune system to promote their malignant growth. One very important mechanism is to change the expression of co-stimulatory and co-inhibitory immune checkpoint molecules on immunocytes. Blocking the signal pathway of immune checkpoint molecules, such as PD1, has been proved to be an extremely promising and effective therapy.

Programmed cell death protein 1 (PD-1), also known as CD279, is a receptor expressed on the surfaces of activated T cells, natural killer T cells, B cells and macrophages. Its structure contains an extracellular domain similar to an immunoglobulin variable region, a transmembrane domain and an intracellular domain, where the intracellular domain contains two phosphorylation sites located in an immunoreceptor tyrosine kinase-based inhibitory domain and an immunoreceptor tyrosine kinase-based transduction domain, suggesting that PD1 can down-regulate T cell receptor-mediated signal pathways.

PD1 has two ligands: PD-L1 and PDL2, and they are different in their expression profiles. The expression of PDL1 will be up-regulated in macrophages and dendritic cells after treatment with lipopolysaccharide (LPS) and granulocyte-macrophage colony-stimulating factor (GM-CSF), and will also be up-regulated in T cells and B cells after stimulation of T cell receptor and B cell receptor signal pathways. PDL1 is also highly expressed in almost all tumor cells, and the expression will be up-regulated after stimulation of interferon (IFN) gamma. As a matter of fact, the expression of PDL1 in a variety of tumors is considered to have prognostic relevance; but, the expression of PD-L2 is relatively concentrated, mainly on dendritic cells.

When T cells expressing PD-1 come into contact with cells expressing the ligands of PD-1, those antigen-stimulated functional activities, such as cell proliferation, cytokine release and cell lysis activity, are all inhibited. Therefore, the interaction between PD1 and ligands thereof can function as an intrinsic negative feedback regulation mechanism to prevent T cell hyperactivation during infection, immune tolerance or tumorigenesis, thus reducing the occurrence of autoimmune diseases and promoting self tolerance. Long-term antigenic stimulation, e.g., in tumor or long-term infection, will cause T cells to express high level of PD-1, gradually lose activities in response to these long-term antigens, and eventually become nonfunctional, namely, the so-called T cell exhaustion. B cells also have the inhibitory effect caused by PD1 and ligands thereof and corresponding functional exhaustion.

Some evidence from preclinical animal studies has indicated that PD-1 and ligands thereof can down-regulate the immunoreaction. PD-1-deficient mice will develop lupus erythematosus-like acute proliferative glomerulonephritis and dilated cardiomyopathy. Utilizing the antibody of PDL1 to block PD-1/PDL1 interaction has been proved to be able to restore and enhance T cell activation in many systems. The monoclonal antibody of PDL1 can also benefit patients with advanced cancers. Some preclinical animal tumor models also show that blocking the signal pathway of PD-1/PD-L1 with a monoclonal antibody can enhance immunoreaction and lead to immunoreactions to a series of histologically different tumors. With the long-term infection LCMV model, the PD-1/PD-L1 interaction has been found to be able to inhibit the activation and proliferation of virus-specific CD8 T cells and the acquisition of effector cell functions. Besides being capable of enhancing the immunoreaction to long-term antigens, blocking the pathway of PD-1/PDL1 has also been found to be able to enhance response to vaccines, including response to a therapeutic vaccine in long-term infection.

To sum up, if, besides the existing monoclonal antibodies, a compound for blocking the PD-1/PD-L1 interaction can be developed, it can serve as an effective therapeutic means for blocking the PD-1/PDL1-mediated inhibitory signal pathway to enhance or restore the function of T cells. Therefore, the compound specifically blocking the PD-1/PD-L1 interaction will achieve a good therapeutic effect in immunotherapies for a variety of cancers and other immunity-related diseases.

### SUMMARY

The object of the present invention is to provide an overall holosymmetric biphenyl derivative, and a preparation method therefor and use thereof, so that a new generation of PD-1/PD-L1 inhibitors are expected to be developed.

A first aspect of the present invention provides a compound of formula (I), a stereoisomer or pharmaceutically acceptable salt thereof:

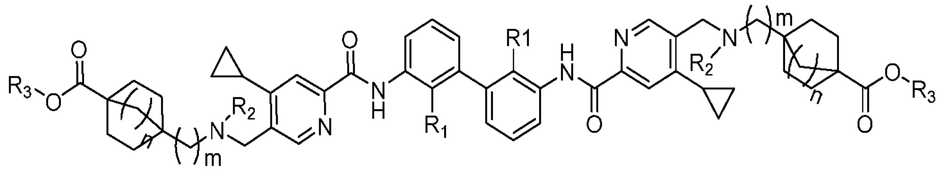

(I)

wherein, both $R_1$ are identical or different and are each independently selected from the group consisting of hydrogen, deuterium, halogen, cyano, $C_{1-4}$ alkyl and $C_{3-6}$ cycloalkyl, the $C_{1-4}$ alkyl and $C_{3-6}$ cycloalkyl are further optionally substituted by one or more substituents selected from the group consisting of deuterium, halogen, cyano, $C_{1-4}$ alkyl, $C_{1-4}$ haloalkyl, $C_{1-4}$ deuterioalkyl, cyclopropyl, hydroxy and $C_{1-4}$ alkoxy;

both $R_2$ are identical or different and are each independently selected from the group consisting of hydrogen, deuterium, $C_{1-4}$ alkyl, $C_{3-6}$ cycloalkyl and hydroxy, the $C_{1-4}$ alkyl and $C_{3-6}$ cycloalkyl are further optionally substituted by one or more substituents selected from the group consisting of deuterium, halogen, cyano, $C_{1-4}$ alkyl, $C_{1-4}$ haloalkyl, $C_{1-4}$ deuterioalkyl, cyclopropyl, hydroxy and $C_{1-4}$ alkoxy;

both $R_3$ are identical or different and are each independently selected from the group consisting of hydrogen, deuterium, $C_{1-10}$ alkyl, $C_{3-10}$ cycloalkyl and 3-10 membered heterocyclyl, the $C_{1-10}$ alkyl, $C_{3-10}$ cycloalkyl and 3-10 membered heterocyclyl are further optionally substituted by one or more substituents selected from the group consisting of deuterium, halogen, cyano, hydroxy, carboxyl, $C_{1-4}$ alkyl, $C_{1-4}$ haloalkyl, $C_{1-4}$ deuterioalkyl, $C_{3-6}$ cycloalkyl, 3-6 membered heterocyclyl, $C_{6-10}$ aryl, 5-8 membered heteroaryl, $C_{3-6}$ cycloalkoxy, 3-6 membered heterocycloxy and $C_{1-4}$ alkoxy;

both m are identical or different and are each independently 0, 1, 2, 3 or 4;

both n are identical or different and are each independently 0, 1, 2, 3 or 4.

As a preferred embodiment, in the compound of formula (I), the stereoisomer or pharmaceutically acceptable salt thereof, both $R_1$ are identical or different and are each independently selected from the group consisting of hydrogen, deuterium, halogen, cyano, $C_{1-4}$ alkyl, $C_{1-4}$ haloalkyl, $C_{1-4}$ deuterioalkyl, cyclopropyl-substituted $C_{1-4}$ alkyl and $C_{3-6}$ cycloalkyl.

As a further preferred embodiment, in the compound of formula (I), the stereoisomer or pharmaceutically acceptable salt thereof, both $R_1$ are identical or different and are each independently selected from the group consisting of hydrogen, deuterium, fluoro, chloro, cyano, methyl, ethyl, isopropyl, trifluoromethyl, trideuteriomethyl, cyclopropylmethyl and cyclopropyl.

As a preferred embodiment, in the compound of formula (I), the stereoisomer or pharmaceutically acceptable salt thereof, both $R_2$ are identical or different and are each independently selected from the group consisting of hydrogen, deuterium, $C_{1-4}$ alkyl, $C_{1-4}$ haloalkyl, $C_{1-4}$ deuterioalkyl, cyclopropyl-substituted $C_{1-4}$ alkyl, $C_{3-6}$ cycloalkyl and hydroxy.

As a further preferred embodiment, in the compound of formula (I), the stereoisomer or pharmaceutically acceptable salt thereof, both $R_2$ are identical or different and are each independently selected from the group consisting of hydrogen, deuterium, methyl, ethyl, isopropyl, trifluoromethyl, trideuteriomethyl, cyclopropylmethyl, cyclopropyl and hydroxy.

As a preferred embodiment, in the compound of formula (I), the stereoisomer or pharmaceutically acceptable salt thereof, both $R_3$ are identical or different and are each independently selected from the group consisting of hydrogen, deuterium, $C_{1-4}$ alkyl, $C_{3-6}$ cycloalkyl and 3-6 membered heterocyclyl, the $C_{1-4}$ alkyl, $C_{3-6}$ cycloalkyl and 3-6 membered heterocyclyl are further optionally substituted by one or more substituents selected from the group consisting of deuterium, halogen, cyano, hydroxy, carboxyl, $C_{1-4}$ alkyl, $C_{1-4}$ haloalkyl, $C_{1-4}$ deuterioalkyl, $C_{3-6}$ cycloalkyl, 3-6 membered heterocyclyl, $C_{3-6}$ cycloalkoxy and $C_{1-4}$ alkoxy.

As a further preferred embodiment, in the compound of formula (I), the stereoisomer or pharmaceutically acceptable salt thereof, both $R_3$ are identical or different and are each independently selected from the group consisting of hydrogen, deuterium, methyl, ethyl, isopropyl, cyclopropyl, cyclobutyl, cyclopentyl, oxacyclobutyl and azacyclobutyl, the methyl, ethyl, isopropyl, cyclopropyl, cyclobutyl, oxacyclobutyl and azacyclobutyl are further optionally substituted by one or more substituents selected from the group consisting of deuterium, halogen, cyano, hydroxy, carboxyl, methyl, ethyl, isopropyl, trifluoromethyl, trideuteriomethyl, cyclopropyl, cyclobutyl, oxacyclobutyl, azacyclobutyl, cyclopropyloxy, cyclobutyloxy, methoxy, ethoxy and isopropoxy.

As a more further preferred embodiment, in the compound of formula (I), the stereoisomer or pharmaceutically acceptable salt thereof, both $R_3$ are identical or different and are each independently selected from the group consisting of hydrogen, deuterium, methyl, ethyl, isopropyl, trifluoromethyl, trideuteriomethyl, cyclopropylmethyl, cyclopropyl, cyclobutyl, oxacyclobutyl and azacyclobutyl.

As a more further preferred embodiment, in the compound of formula (I), the stereoisomer or pharmaceutically

acceptable salt thereof, both $R_1$ are identical and are selected from the group consisting of hydrogen, deuterium, fluoro, chloro, cyano, methyl, ethyl, isopropyl, trifluoromethyl, trideuteriomethyl, cyclopropylmethyl and cyclopropyl;

both $R_2$ are identical and are selected from the group consisting of hydrogen, deuterium, methyl, ethyl, isopropyl, trifluoromethyl, trideuteriomethyl, cyclopropylmethyl, cyclopropyl and hydroxy;
both $R_3$ are identical and are selected from the group consisting of hydrogen, deuterium, methyl, ethyl, isopropyl, trifluoromethyl, trideuteriomethyl, cyclopropylmethyl, cyclopropyl, cyclobutyl, oxacyclobutyl and azacyclobutyl;
both m are identical and are 0, 1 or 2;
both n are identical and are 1 or 2.

As the most preferred embodiment, the compound of formula (I), the stereoisomer or pharmaceutically acceptable salt thereof include, but are not limited to, the following compounds:

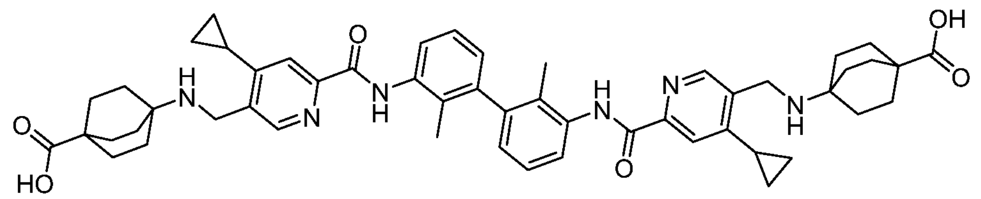

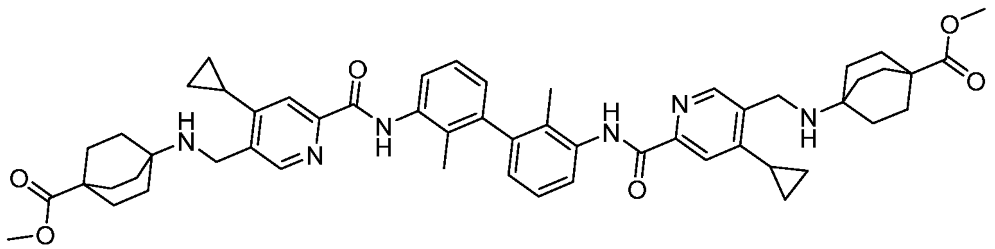

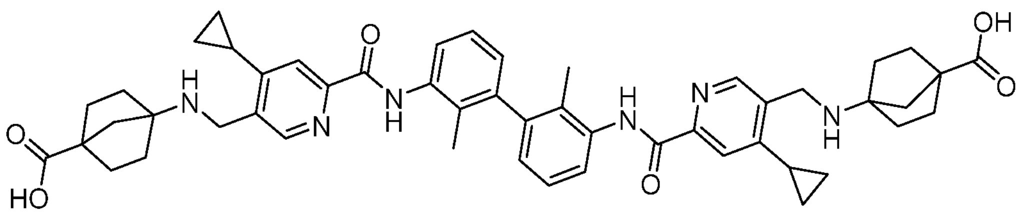

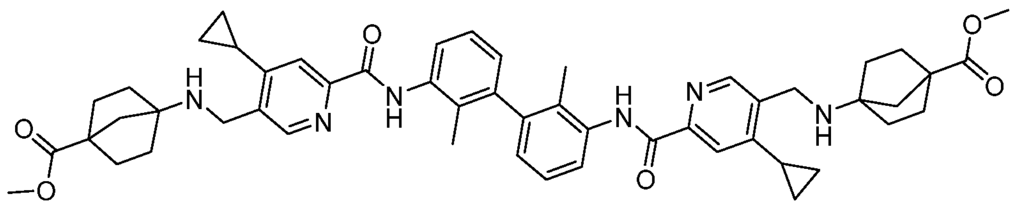

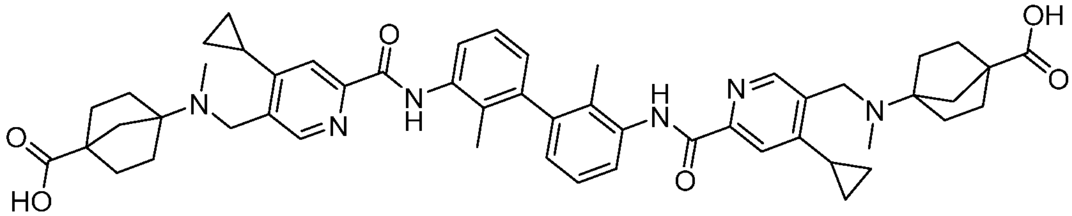

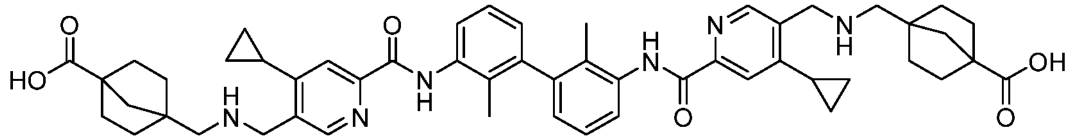

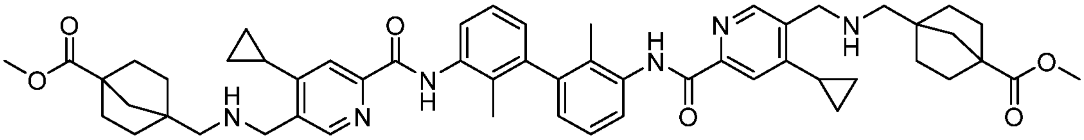

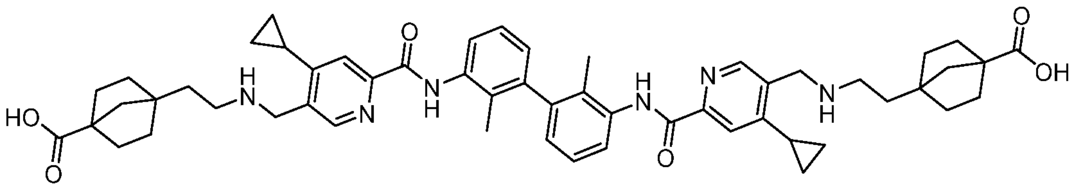

and

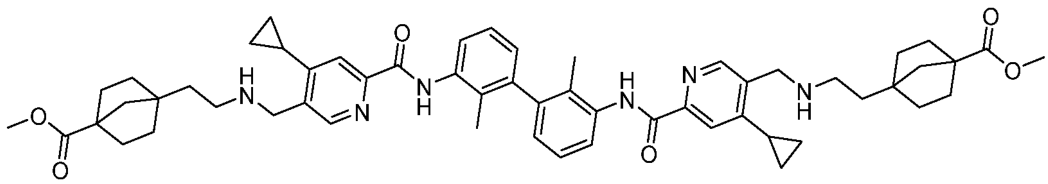

.

A second aspect of the present invention provides a pharmaceutical composition comprising the compound of formula (I), the stereoisomer or pharmaceutically acceptable salt thereof described above and a pharmaceutically acceptable carrier.

A third aspect of the present invention provides use of the compound of formula (I), the stereoisomer or pharmaceutically acceptable salt thereof in the preparation of a medicament for treating a PD-1/PD-L1 signal pathway-mediated disease.

As a preferred embodiment, the PD-1/PD-L1 signal pathway-mediated disease is cancer or tumor, immune-related disease and disorder, communicable disease, infectious disease or metabolic disease.

As a further preferred embodiment, the cancer or tumor is lymphoma (including but not limited to lymphocytic lymphoma, primary central nervous system lymphoma, T cell lymphoma, diffuse large B cell lymphoma, follicle center lymphoma, Hodgkin lymphoma, non-Hodgkin lymphoma or primary mediastinal large B cell lymphoma), sarcoma (including but not limited to Kaposi's sarcoma, fibrosarcoma, liposarcoma, chondrosarcoma, osteosarcoma, leiomyosarcoma, rhabdomyosarcoma, soft tissue sarcoma, angiosarcoma or lymphangiosarcoma), melanoma, glioblastoma, synovioma, meningioma, biliary tract tumor, thymic tumor, neuroma, seminoma, nephroblastoma, pleomorphic adenoma, hepatocellular papilloma, renal tubule adenoma, cystadenoma, papilloma, adenoma, leiomyoma, rhabdomyoma, hemangioma, lymphangioma, osteoma, chondroma, lipoma, fibroma, central nervous system tumor, rhachiophyma, brain stem glioma, pituitary adenoma, multiple myeloma, ovarian tumor, myelodysplastic syndrome or mesothelioma, prostate cancer, recurrent prostate cancer or prostate cancer having developed resistance to existing medicaments, thyroid cancer, parathyroid cancer, anal cancer, testicular cancer, urethral carcinoma, penile cancer, bladder cancer, ureteral cancer, uterine cancer, ovarian cancer, fallopian tube cancer, endometrial cancer, cervical cancer, vaginal cancer, vulvar cancer, adrenal cancer, Merkel cell carcinoma, embryonal carcinoma, chronic or acute leukemia (including but not limited to acute myeloid leukemia, chronic myeloid leukemia, acute lymphoblastic leukemia, chronic granulocytic leukemia and chronic lymphoblastic leukemia), bronchial carcinoma, esophageal cancer, nasopharyngeal carcinoma, hepatocellular carcinoma, renal cell carcinoma, small cell lung cancer, basal cell carcinoma, lung cancer, breast cancer, adenocarcinoma, papillary carcinoma, cystadenocarcinoma, squamous non-small cell lung cancer, non-squamous non-small cell lung cancer, rectal cancer, colon cancer, colorectal cancer, gastric cancer, pancreatic cancer, head and neck squamous cell carcinoma, head and neck cancer, gastrointestinal cancer, bone cancer, skin cancer, small intestine cancer, endocrine cancer, renal pelvic carcinoma, epidermoid carcinoma, abdominal wall carcinoma, renal cell carcinoma, transitional cell carcinoma, choriocarcinoma or metastatic tumor;

the immune-related disease and disorder is rheumatic arthritis, renal failure, lupus erythematosus, asthma, psoriasis, ulcerative colitis, pancreatitis, allergy, fibrosis, anemia, fibromyalgia, Alzheimer's disease, congestive heart failure, stroke, aortic valve stenosis, arteriosclerosis, osteoporosis, Parkinson's disease, infection, Crohn's disease, ulcerative colitis, allergic contact dermatitis and eczema, systemic sclerosis or multiple sclerosis;
the infectious disease is bacterial infectious disease, viral infectious disease or fungal infectious disease;
the communicable disease or infectious disease is sepsis, liver infection, HIV, hepatitis A, hepatitis B, hepatitis C, hepatitis D, herpes virus, papillomavirus or influenza;
the metabolic disease is diabetes, diabetic ketoacidosis, hyperglycemic hyperosmolar syndrome, hypoglycemia, gout, malnutrition, vitamin A deficiency, scurvy, vitamin D deficiency or osteoporosis.

In a fourth aspect, the present invention provides the compound of formula (I), the stereoisomer or pharmaceutically acceptable salt thereof for use as a medicament for treating PD-1/PD-L1 signal pathway-mediated cancer or tumor, immune-related disease and disorder, communicable disease, infectious disease or metabolic disease.

The present invention also relates to a method for treating PD-1/PD-L1 signal pathway-mediated cancer or tumor, immune-related disease and disorder, communicable disease, infectious disease or metabolic disease, which comprises administering to a patient in need thereof a clinically effective amount of the compound of formula (I), the stereoisomer or pharmaceutically acceptable salt thereof.

## DETAILED DESCRIPTION

After extensive and intensive research, the inventors of the present invention, for the first time, developed a biphenyl series of compounds having the structure of general formula (I). The series of compounds of the present invention have a strong inhibitory effect on the PD-1/PD-L1 interaction, and they can be widely used in the preparation of a drug for preventing and/or treating PD-1/PD-L1 signal pathway-mediated cancer or tumor, immune-related disease and disorder, communicable disease, infectious disease or metabolic disease and are expected to be developed into a new generation of PD-1/PD-L1 inhibitors. The present invention is achieved on this basis.

Detailed description: unless otherwise stated or specified, the following terms used in the specification and claims have the following meanings.

"Alkyl" refers to linear or branched saturated aliphatic alkyl groups, preferably linear alkyl or branched alkyl containing 1 to 10 or 1 to 4 carbon atoms, including but not limited to methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, and the like. "$C_{1-10}$ alkyl" refers to linear alkyl and branched alkyl containing 1 to 10 carbon atoms, and "$C_{1-4}$ alkyl" refers to linear alkyl and branched alkyl containing 1 to 4 carbon atoms.

Alkyl may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Cycloalkyl" or "carbocycle" refers to a monocyclic or polycyclic hydrocarbon substituent that is saturated or partially unsaturated. The partially unsaturated cyclic hydrocarbon means that the cyclic hydrocarbon may contain one or more (preferably 1, 2 or 3) double bonds, but none of the rings has a fully conjugated $\pi$-electron system; cycloalkyl is classified into monocyclic cycloalkyl and polycyclic cycloalkyl and is preferably cycloalkyl containing 3 to 10 or 3 to 6 carbon atoms. For example, "$C_{3-10}$ cycloalkyl" refers to cycloalkyl containing 3 to 10 carbon atoms, and "$C_{3-6}$ cycloalkyl" refers to cycloalkyl containing 3 to 6 carbon atoms, wherein

monocyclic cycloalkyl includes, but is not limited to, cyclopropyl, cyclobutyl, cyclopentyl, cyclopentenyl, cyclohexyl, cyclohexenyl, cyclohexadienyl, cycloheptyl, cycloheptatrienyl, cyclooctyl and the like;
polycyclic cycloalkyl includes spirocycloalkyl, fused cycloalkyl and bridged cycloalkyl. "Spirocycloalkyl" refers to a polycyclic group in which a carbon atom (called a spiro-atom) is shared among monocyclic rings, wherein those rings may contain one or more (preferably, 1, 2 or 3) double bonds, but none of them has a fully conjugated $\pi$-electron system. According to the number of the spiro-atoms shared among the rings, the spirocycloalkyl may be monospirocycloalkyl, bispirocycloalkyl or polyspirocycloalkyl, including but not limited to:

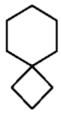 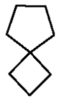 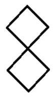 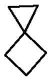 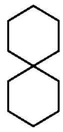 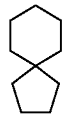 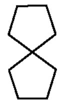 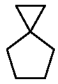.

"Fused cycloalkyl" refers to an all-carbon polycyclic group in which each ring shares a pair of adj acent carbon atoms with the other rings in the system, wherein one or more of the rings may contain one or more (preferably, 1, 2 or 3) double bonds, but none of them has a fully conjugated π-electron system. According to the number of formed rings, the fused cycloalkyl may be bicyclic, tricyclic, tetracyclic or polycyclic, including but not limited to:

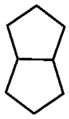 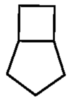 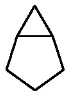 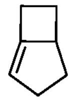 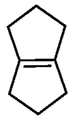

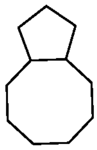 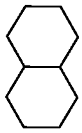 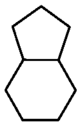 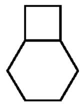 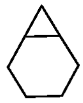.

"Bridged cycloalkyl" refers to an all-carbon polycyclic group in which any two rings share two carbon atoms that are not directly connected to each other, wherein these rings may contain one or more (preferably, 1, 2 or 3) double bonds, but none of them has a fully conjugated π-electron system. According to the number of formed rings, the bridged cycloalkyl may be bicyclic, tricyclic, tetracyclic or polycyclic, including but not limited to:

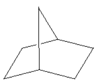 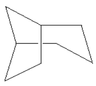 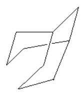 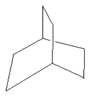 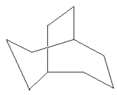 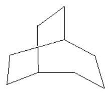 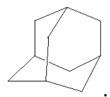.

The cycloalkyl ring can be fused to an aryl, heteroaryl or heterocycloalkyl ring, wherein the ring attached to the parent structure is cycloalkyl, which includes, but is not limited to, indanyl, tetrahydronaphthyl, benzocycloheptyl and the like.

"Cycloalkyl" or "carbocycle" may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Heterocyclyl" or "heterocycle" refers to a monocyclic or polycyclic hydrocarbon substituent that is saturated or partially unsaturated. The partially unsaturated cyclic hydrocarbon means that the cyclic hydrocarbon may contain one or more (preferably 1, 2 or 3) double bonds, but none of the rings has a fully conjugated π-electron system. One or more (preferably 1, 2, 3 or 4) ring atoms in the heterocyclyl are selected from heteroatoms N, O, N•O and $S(O)_r$ (where r is an integer of 0, 1 or 2), excluding ring moiety of -O-O-, -O-S- or -S-S-, and the remaining ring atoms are carbon atoms. Preferred is heterocyclyl comprising 3 to 10 or 3 to 6 ring atoms, e.g. "3-6 membered heterocyclyl" refers to heterocyclyl comprising 3 to 6 ring atoms, and "3-10 membered heterocyclyl" refers to heterocyclyl comprising 3 to 10 ring atoms.

Monocyclic heterocyclyl includes, but is not limited to, pyrrolidinyl, piperidinyl, piperazinyl, morpholinyl, thiomorpholinyl, homopiperazinyl, oxetanyl, tetrahydrofuranyl and the like.

Polycyclic heterocyclyl includes spiroheterocyclyl, fused heterocyclyl and bridged heterocyclyl. "Spiroheterocyclyl" refers to a polycyclic heterocyclyl group in which an atom (called a spiro-atom) is shared among monocyclic rings, wherein one or more (preferably 1, 2, 3 or 4) ring atoms are heteroatoms selected from N, O, N•O and $S(O)_r$ (where r is an integer of 0, 1 or 2), and the remaining ring atoms are carbon atoms. These rings may contain one or more (preferably, 1, 2 or 3) double bonds, but none of them has a fully conjugated π-electron system. According to the number of spiro-atoms shared among the rings, the spiroheterocyclyl may be monospiroheterocyclyl, bispiroheterocyclyl or polyspiroheterocyclyl. Spiroheterocyclyl includes, but is not limited to:

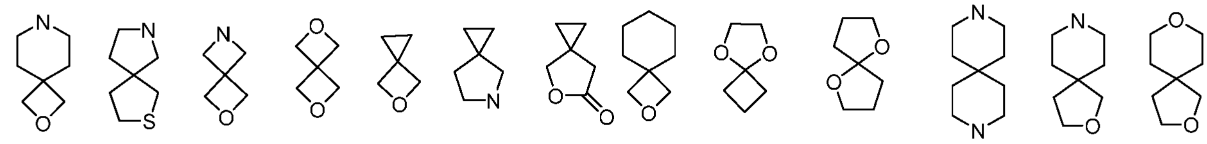

.

"Fused heterocyclyl" refers to a polycyclic heterocyclyl group in which each ring shares a pair of adjacent atoms with the other rings in the system, wherein one or more (preferably, 1, 2, 3 or 4) of the rings may contain one or more (preferably, 1, 2 or 3) double bonds, but none of them has a fully conjugated $\pi$-electron system, wherein one or more (preferably, 1, 2, 3 or 4) ring atoms are heteroatoms selected from N, O, N•O and $S(O)_r$ (where r is an integer of 0, 1 or 2), and the remaining ring atoms are carbon atoms. According to the number of formed rings, the fused heterocyclyl may be bicyclic, tricyclic, tetracyclic or polycyclic, including, but not limited to:

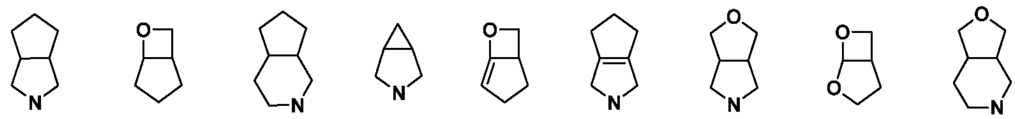

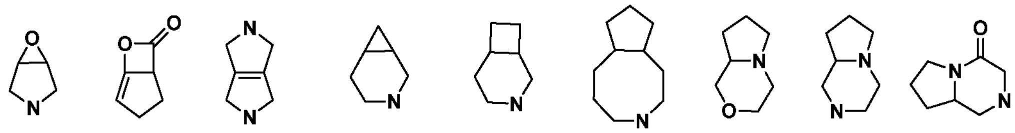

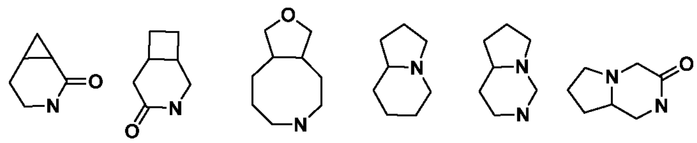

"Bridged heterocyclyl" refers to a polycyclic heterocyclyl group in which any two rings share two carbon atoms that are not directly connected to each other, wherein these rings may contain one or more (preferably, 1, 2 or 3) double bonds, but none of them has a fully conjugated $\pi$-electron system, wherein one or more (preferably, 1, 2, 3 or 4) ring atoms are heteroatoms selected from N, O, N•O and $S(O)_r$ (where r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

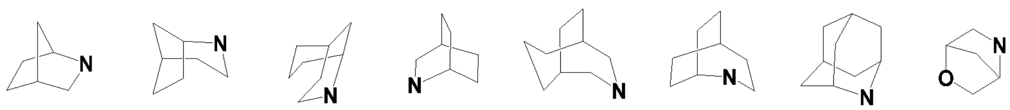

.

The heterocyclyl ring may be fused to an aryl, heteroaryl or cycloalkyl ring, wherein the ring attached to the parent structure is heterocyclyl, including but not limited to:

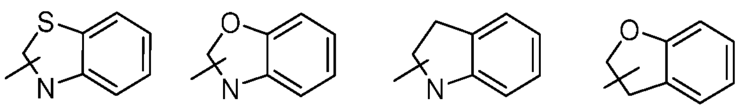

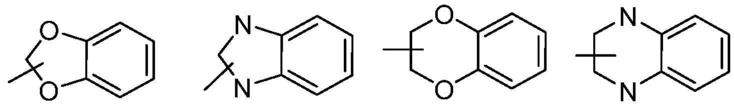

.

"Heterocyclyl" or "heterocycle" may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Aryl" or "aromatic ring" refers to an all-carbon monocyclic or fused-polycyclic (i.e., rings that share a pair of

adjacent carbon atoms) group and a polycyclic (i.e., rings with adjacent pairs of carbon atoms) group having a conjugated $\pi$-electron system, preferably all-carbon aryl containing 6 to 10 carbon atoms; for example, "$C_{6-10}$ aryl" refers to all-carbon aryl containing 6 to 10 carbon atoms, including but not limited to, phenyl and naphthyl. The aryl ring can be fused to a heteroaryl, heterocyclyl or cycloalkyl ring, wherein the ring attached to the parent structure is the aryl ring, including but not limited to:

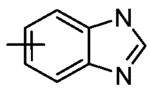 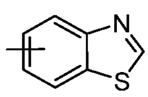 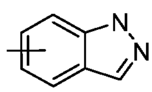 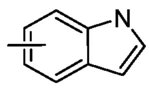 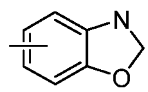

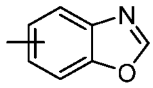 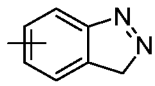 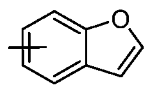 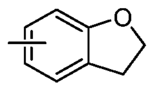 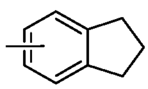

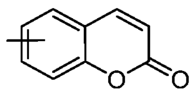 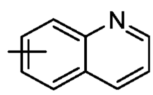 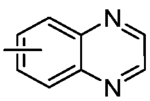 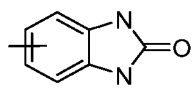 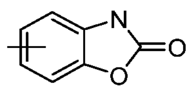 .

"Aryl" or "aromatic ring" may be substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Heteroaryl" or "heteroaromatic ring" refers to a heteroaromatic system containing one or more (preferably 1, 2, 3 or 4) heteroatoms including N, O, N•O and $S(O)_r$ (where r is an integer of 0, 1 or 2), and is preferably a heteroaromatic system containing 5 to 10 or 5 to 8 ring atoms. For example, "5-8 membered heteroaryl" refers to a heteroaromatic system containing 5 to 8 ring atoms, and "5-10 membered heteroaryl" refers to a heteroaromatic system containing 5 to 10 ring atoms. The heteroaryl includes, but is not limited to, furyl, thiophenyl, pyridyl, pyrrolyl, N-alkylpyrrolyl, pyrimidinyl, pyrazinyl, imidazolyl, tetrazolyl and the like. The heteroaryl ring can be fused to an aryl, heterocyclyl or cycloalkyl ring, wherein the ring attached to the parent structure is the heteroaryl ring, including but not limited to:

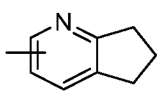 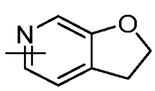 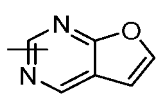 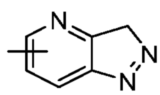 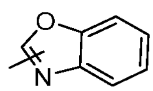

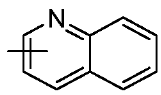 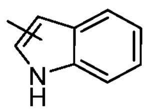 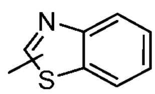 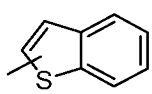 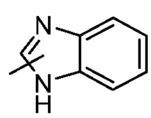 .

"Heteroaryl" or "heteroaryl ring" may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Alkenyl" refers to alkyl defined as above consisting of at least two carbon atoms and at least one carbon-carbon double bond, and is preferably linear or branched alkenyl containing 2 to 10 carbon atoms. For example, "$C_{2-10}$ alkenyl" refers to linear or branched alkenyl containing 2 to 10 carbon atoms. Alkenyl includes, but is not limited to, vinyl, 1-propenyl, 2-propenyl, 1-, 2- or 3-butenyl, and the like.

"Alkenyl" may be substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Alkynyl" refers to alkyl defined as above consisting of at least two carbon atoms and at least one carbon-carbon triple bond, and is preferably linear or branched alkynyl containing 2 to 10 carbon atoms. For example, "$C_{2-10}$ alkynyl" refers to linear or branched alkynyl containing 2 to 10 carbon atoms. Alkynyl includes, but is not limited to, ethynyl, 1-propynyl, 2-propynyl, 1-, 2- or 3-butynyl, and the like.

"Alkynyl" may be substituted or unsubstituted, and when it is substituted, the substituents are preferably one or

more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Alkoxy" refers to -O-(alkyl), wherein the alkyl is defined as above. For example, "$C_{1-4}$ alkoxy" refers to alkoxy containing 1 to 4 carbons atoms. Alkoxy includes, but is not limited to, methoxy, ethoxy, propoxy, butoxy, and the like.

"Alkoxy" may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Cycloalkoxy" or "cycloalkyloxy" refers to -O-cycloalkyl, wherein the cycloalkyl is defined as above. For example, "$C_{3-6}$ cycloalkoxy" refers to cycloalkyloxy containing 3 to 6 carbon atoms. Cycloalkoxy or cycloalkyloxy includes, but is not limited to, cyclopropyloxy, cyclobutyloxy, cyclopentyloxy, cyclohexyloxy, etc.

"Cycloalkoxy" or "cycloalkyloxy" may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"Heterocycloxy" or "heterocyclyloxy" refers to -O-heterocyclyl, wherein the heterocyclyl is defined as above, and heterocyoxy or heterocyclyloxy includes, but is not limited to, azacyclobutyloxy, oxacyclobutyloxy, azacyclopentyloxy, nitrogen, oxacyclohexyloxy and the like.

"Heterocycloxy" or "heterocyclyloxy" may be optionally substituted or unsubstituted, and when it is substituted, the substituents are preferably one or more (preferably 1, 2, 3 or 4) groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-10}$ cycloalkyl, 3-10 membered heterocyclyl, $C_{6-10}$ aryl, 5-10 membered heteroaryl, =O, =S and $-SF_5$.

"$C_{1-4}$ haloalkyl" refers to an alkyl group having 1 to 4 carbon atoms in which hydrogen on the alkyl is optionally substituted with a fluorine, chlorine, bromine or iodine atom, and includes, but is not limited to, difluoromethyl, dichloromethyl, dibromomethyl, trifluoromethyl, trichloromethyl, tribromomethyl and the like.

"$C_{1-4}$ deuterioalkyl" refers to an alkyl group having 1 to 4 carbon atoms in which hydrogen on the alkyl is optionally substituted with a deuterium atom, and includes, but is not limited to, monodeuteriomethyl, dideuteriomethyl, trideuteriomethyl and the like.

"Halogen" refers to fluoro, chloro, bromo or iodo, "NaOH" refers to sodium hydroxide, "$BH_3$•$Me_2S$" refers to borane dimethylsulfide, "MeOH" refers to methanol, "$NaBH_3CN$" refers to sodium cyanoborohydride, "HOAc" refers to acetic acid, "DMF" refers to *N,N*-dimethylformamide, and "THF" refers to tetrahydrofuran.

The term "optional" or "optionally" means that the event or circumstance subsequently described may, but not necessarily, occur, and that the description includes instances where the event or circumstance occurs or does not occur, that is, instances where substitution occurs or does not occur. For example, "heterocyclyl group optionally substituted with alkyl" means that alkyl may be, but not necessarily, present, and that the description includes instances where the heterocyclyl group is or is not substituted with alkyl.

The term "substituted" means that one or more "hydrogen atoms" in the group are each independently substituted by a corresponding number of substituents. It goes without saying that a substituent is only in its possible chemical position and consistent with chemical valence bond theory, and those skilled in the art will be able to determine (by studies or theories) possible or impossible substitution without undue efforts. For example, it may be unstable when amino or hydroxy having free hydrogen is bound to a carbon atom having an unsaturated bond (such as olefin).

"Stereoisomers" refer to isomers produced by different spatial arrangements of atoms in molecules, and can be classified into *cis-trans* isomers and enantiomers, and also into enantiomers and diastereomers. Stereoisomers resulting from rotation of single bonds are referred to as conformational stereo-isomers and sometimes also as rotamers. Stereoisomers resulting from bond lengths, bond angles, intramolecular double bonds, rings and the like are referred to as configuration stereo-isomers, and the configuration stereo-isomers are classified into two categories. Among them, isomers resulting from the fact that a double bond or a single bond of a ring-forming carbon atom cannot rotate freely are referred to as geometric isomers and also as *cis-trans* isomers, and the isomers are classified into *Z* and *E* configurations. For example, *cis*-2-butene and *trans*-2-butene are a pair of geometric isomers, and stereoisomers having different optical rotation properties due to the absence of anti-axisymmetry in the molecule are referred to as optical isomers, and are classified into *R* and *S* configurations. In the present invention, the term "stereoisomer" is understood to include one or more of the above enantiomers, configuration stereo-isomers and conformational stereo-isomers, unless otherwise specified.

"Pharmaceutically acceptable salt" used herein refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example,

physiologically/pharmaceutically acceptable carriers and excipients. The purpose of the pharmaceutical composition is to promote the administration to an organism, which facilitates the absorption of the active ingredient, thereby exerting biological activities.

**The present invention is further explained in detail below with reference to examples, which are not intended to limit the present invention, and the present invention is not merely limited to the contents of the examples.**

The compound structure of the present invention is determined by nuclear magnetic resonance (NMR) and/or liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-$d_6$), tetradeuteromethanol ($CD_3OD$), and deuterated chloroform ($CDCl_3$) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatograph (Sunfire C18 150 * 4.6 mm chromatographic column) and a Waters 2695-2996 high pressure liquid chromatograph (Gimini C18 150 * 4.6 mm chromatographic column).

A Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for product separation and purification is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, the solvent is a dry solvent, and the reaction temperature is in degree centigrade (°C).

**Preparation of Compounds of Specific Examples**

**Example 1: preparation of 4-(((6-((3'-(5-(((4-carboxybicyclo[2.2.2]octan-1-yl)amino)met hyl)-4-cyclopropylpicolinamido)-2,2'-dimethyl-[1,1'-biphenyl]-3-yl)carbamoyl)-4-cyclopropylpyridin-3-yl)methyl)amino)bicyclo[2.2.2] octane-1-carboxylic acid**

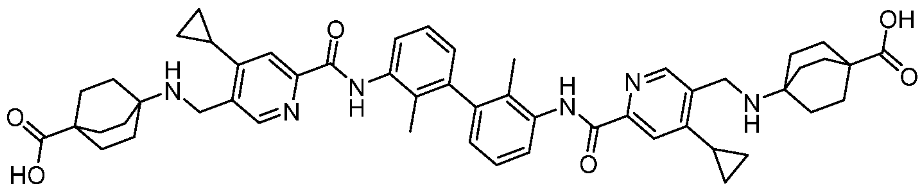

**Step 1: synthesis of methyl 4-(((4-cyclopropyl-6-((3'-(4-cyclopropyl-5-(((4-(methoxycarbonyl)bicyclo[2.2.2]octan-1-yl)amino)methyl)picolinamido)-2,2'-dimethyl-[1,1'-biphenyl]-3-yl)carbamoyl)pyridin-3-yl)methyl)amino) bicyclo[2.2.2]octane-1-carboxylate**

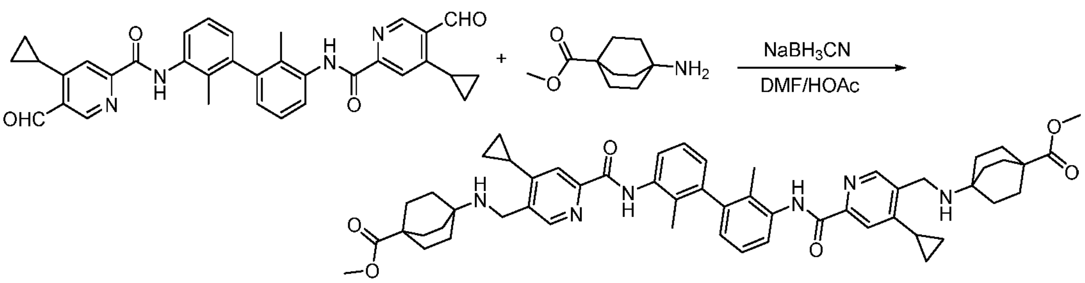


*N,N'*-(2,2'-dimethyl-[1,1'-biphenyl]-3,3'-diyl)bis(4-cyclopropyl-5-formylpicolinamide) [pre pared according to the synthesis method in WO2021008491A1] (200 mg, 0.358 mmol) was placed in DMF (5 mL), followed by addition of methyl

4-aminobicyclo[2.2.2]octane-1-carboxylate (656.1 mg, 3.58 mmol) and HOAc (1.5 mL). The mixture was stirred at room temperature for 1 hr. Sodium cyanoborohydride (225.0 mg, 3.58 mmol) was added, and the mixture was stirred overnight at room temperature. After the reaction was completed, the residue was separated by reversed-phase column chromatography to obtain methyl 4-(((4-cyclopropyl-6-((3'-(4-cyclopropyl-5-(((4-(methoxycarbonyl)bicyclo[2.2.2]octan-1-yl)amino)methyl)picolinamido)-2,2'-dimethyl-[1,1'-biphenyl]-3-yl)carbamoyl)pyridin-3-yl)methyl)amino)bicyclo[2.2.2]octane-1-carboxylate (180 mg, 0.20 mmol, yield: 56.3%). MS m/z (ESI): 893.6 $[M+H]^+$.

**Step 2: synthesis of 4-(((6-((3'-(5-(((4-carboxybicyclo[2.2.2]octan-1-yl)amino)methyl)-4-cyclopropylpicolinamido)-2,2'-dimethyl-[1,1'-biphenyl]-3-yl)carbamoyl)-4-cyclopropylpyridin-3-yl)methyl)amino)bicyclo[2.2.2]octane-1-carboxylic acid**

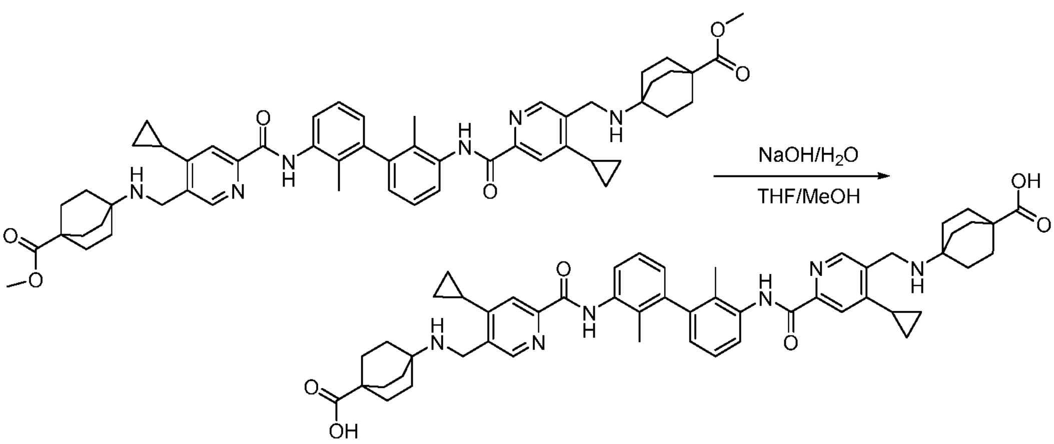


Methyl 4-(((4-cyclopropyl-6-((3'-(4-cyclopropyl-5-(((4-(methoxycarbonyl)bicyclo[2.2.2]octan-1-yl)amino)methyl)picolinamido)-2,2'-dimethyl-[1,1'-biphenyl]-3-yl)carbamoyl)pyridin-3-yl)methyl)amino)bicyclo[2.2.2]octane-1-carboxylate(350 mg, 0.39 mmol) was placed in a mixture solution of MeOH (5 mL), THF (5 mL) and water (5 mL), followed by addition of NaOH (314 mg, 7.84 mmol). The mixture was heated to 50 °C and stirred overnight under nitrogen protection. After the reaction was completed, the reaction solution was adjusted to pH of ~6 with formic acid and then concentrated. The crude product was separated by reversed-phase column chromatography to obtain 4-(((6-((3'-(5-(((4-carboxybicyclo[2.2.2]octan-1-yl)amino)methyl)-4-cyclopropylpicolinamido)-2,2'-dimethyl-[1,1'-biphenyl]-3-yl)carbamoyl)-4-cyclopropylpyridin-3-yl)methyl)amino)bicyclo[2.2.2]octane-1-carboxylic acid (180 mg, 0.187 mmol, yield: 47.8%). MS m/z (ESI): 865.6 $[M+H]^+$.

$^1$H NMR (400 MHz, DMSO-$d_6$) δ 10.31 (s, 2H), 8.55 (s, 2H), 7.89 (d, $J$ = 8.0 Hz, 2H), 7.57 (s, 2H), 7.31 (t, $J$ = 7.8 Hz, 2H), 6.97 (d, $J$ = 7.5 Hz, 2H), 3.87 (s, 4H), 2.28 (td, $J$ = 8.3, 4.2 Hz, 2H), 2.00 (s, 6H), 1.77 (dd, $J$ = 10.5, 5.3 Hz, 12H), 1.60 (dd, $J$ = 10.3, 5.5 Hz, 12H), 1.12 (dt, $J$ = 7.7, 4.9 Hz, 4H), 0.84 (q, $J$ = 5.2 Hz, 4H).

**Example 2: preparation of 4,4'-((((((2,2'-dimethyl-[1,1'-biphenyl]-3,3'-diyl)bis(azanediyl))bis (carbonyl))bis(4-cyclopropylpyridine-6,3-diyl))bis(methylene))bis(azanediyl))bis(bicyclo[2.2.1]heptane-1-carboxylic acid)**

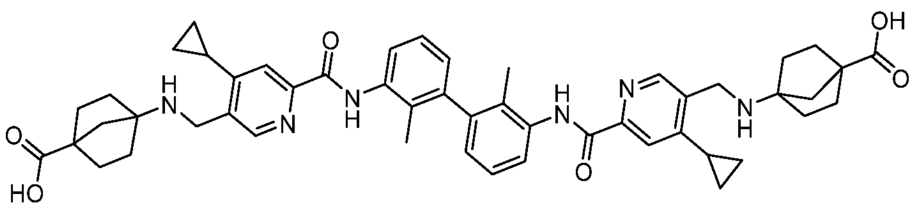

**Step 1: synthesis of 4-(methoxycarbonyl)bicyclo[2.2.1]heptane-1-carboxylic acid**

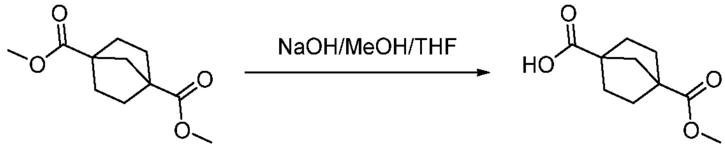


Dimethyl bicyclo[2.2.1]heptane-1,4-dicarboxylate (2.0 g, 9.42mmol) was dissolved in THF (40 mL), and a solution of NaOH (377 mg, 9.42 mmol) in methanol (8 mL) was added dropwise. The mixture was stirred overnight at room temperature. After the reaction was completed, the reaction solution was carefully concentrated until the solvent was completely removed. The solid was washed with petroleum ether and filtered, and the filter cake was dissolved in water (50 mL). The aqueous solution was acidified with 2 M hydrochloric acid until the pH value was ~4 and then extracted with ethyl acetate. The organic phase was washed with water and saturated brine, dried over anhydrous sodium sulfate and filtered, and the filtrate was concentrated to obtain 4-(methoxycarbonyl)bicyclo[2.2.1]heptane-1-carboxylic acid (1.49 g, yield: 80.0%).

**Step 2: synthesis of methyl 4-(((benzyloxy)carbonyl)amino)bicyclo[2.2.1]heptane-1-carboxylate**

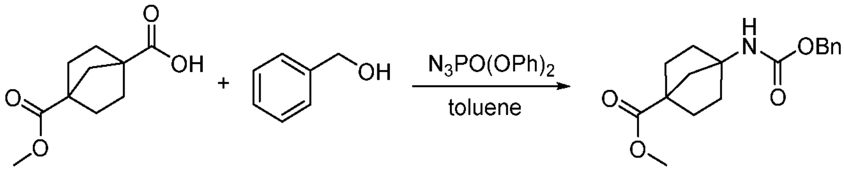


4-(methoxycarbonyl)bicyclo[2.2.1]heptane-1-carboxylic acid (700 mg, 3.53 mmol) was dissolved in toluene (30 mL), followed by addition of diphenylphosphoryl azide (0.91 mL, 4.24 mmol) and triethylamine (0.98 mL, 7.06 mmol). The mixture was heated to 120 °C and reacted for 2 hrs under nitrogen protection. The reaction solution was cooled to room temperature, and then benzyl alcohol (0.73 mL, 7.06 mmol) was added. The mixture was reacted at 100 °C for 18 hrs under nitrogen protection. After the reaction was completed, the reaction solution was concentrated, and the crude product was separated by column chromatography to obtain methyl 4-(((benzyloxy)carbonyl)amino)bicyclo[2.2.1]heptane-1-carboxylate (1000 mg, 3.30 mmol, yield: 93.4%). MS m/z (ESI): 304.0 $[M+H]^+$.

**Step 3: synthesis of methyl 4-aminobicyclo[2.2.1]heptane-1-carboxylate**

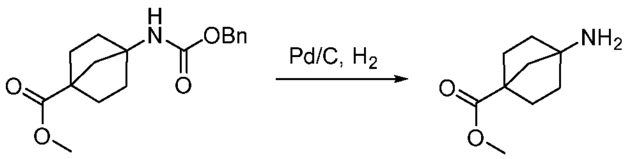


Methyl 4-(((benzyloxy)carbonyl)amino)bicyclo[2.2.1]heptane-1-carboxylate (1000 mg, 3.30 mmol) was dissolved in methanol (30 mL), followed by addition of 10% Pd/C (100 mg). The mixture was stirred overnight at room temperature under atmospheric hydrogen atmosphere. After the reaction was completed, the reaction solution was filtered, and the filtrate was concentrated to obtain methyl 4-aminobicyclo[2.2.1]heptane-1-carboxylate (500 mg, 2.955 mmol, yield: 89.6%). MS m/z (ESI): 170.0 $[M+H]^+$.

**Step 4: synthesis of dimethyl 4,4'-((((((2,2'-dimethyl-[1,1'-biphenyl]-3,3'-diyl)bis(azanediyl))bis(carbonyl)) bis(4-cyclopropylpyridine-6,3-diyl))bis(methylene))bis(azanediyl))bis(bicyclo[2.2.1]heptane-1-carboxylate)**

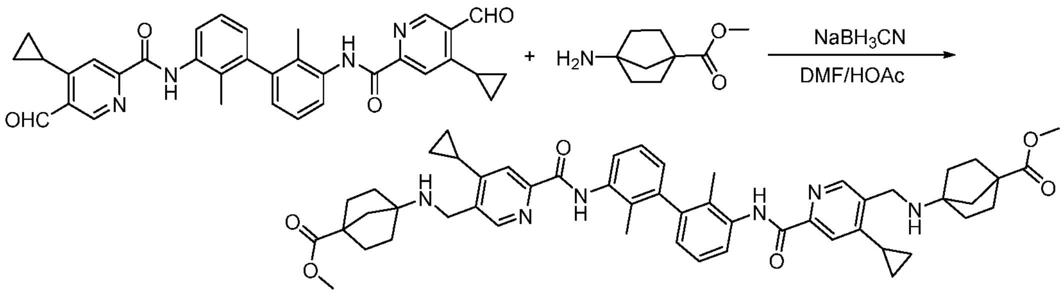


*N*,*N'*-(2,2'-dimethyl-[1,1'-biphenyl]-3,3'-diyl)bis(4-cyclopropyl-5-formylpicolinamide) (200 mg, 0.358 mmol) was dissolved in DMF (5 mL), followed by addition of methyl 4-aminobicyclo[2.2.1]heptane-1-carboxylate (484.7 mg, 2.864 mmol) and HOAc (1.5 mL). The mixture was stirred at room temperature for 60 min, and then $NaBH_3CN$ (225.0 mg, 3.58 mmol) was added. The resulting mixture was stirred overnight at room temperature under nitrogen protection. After the reaction was completed, the reaction solution was separated by column chromatography (water/acetonitrile/formic acid) to obtain dimethyl 4,4'-((((((2,2'-dimethyl-[1,1'-biphenyl]-3,3'-diyl)bis(azanediyl))bis(carbonyl)) bis(4-cyclopropylpyridine-6,3-diyl))bis(methylene))bis(azanediyl))bis(bicyclo[2.2.1]heptane-1-carboxylate)
(230 mg, 0.266 mmol, yield: 74.3%). MS m/z (ESI): 865.4 $[M+H]^+$.

**Step 5: synthesis of 4,4'-((((((2,2'-dimethyl-[1,1'-biphenyl]-3,3'-diyl)bis(azanediyl))bis (carbonyl))bis(4-cyclopropylpyridine-6,3-diyl))bis(methylene))bis(azanediyl))bis(bicyclo[2.2.1]heptane-1-carboxylic acid)**

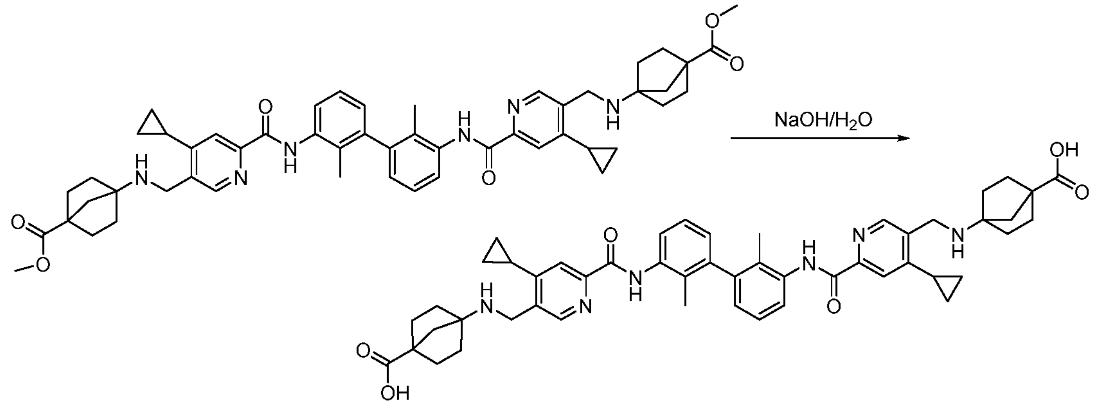


Dimethyl 4,4'-((((((2,2'-dimethyl-[1,1'-biphenyl]-3,3'-diyl)bis(azanediyl))bis(carbonyl)) bis(4-cyclopropylpyridine-6,3-diyl))bis(methylene))bis(azanediyl))bis(bicyclo[2.2.1]heptane-1-carboxylate) (230 mg, 0.266 mmol) was added to a mixture solution of MeOH (5 mL), THF (5 mL) and water (5 mL), followed by addition of NaOH (212.7 mg, 5.317 mmol). The mixture was heated to 50 °C and stirred for 1 hr under nitrogen protection. After the reaction was completed, the reaction solution was adjusted to pH of ~6 with formic acid and then concentrated. The crude product was separated by reversed-phase column chromatography to obtain 4,4'-((((((2,2'-dimethyl-[1,1'-biphenyl]-3,3'-diyl)bis(azanediyl))bis(carbonyl))bis(4-cyclopropylpyridine-6,3-diyl))bis(methylene))bis(azanediyl))bis(bicyclo[2.2.1]heptane-1-carboxylic acid) (160 mg, 0.181 mmol, yield: 67.9%). MS m/z (ESI): 837.4 $[M+H]^+$.

$^1H$ NMR (400 MHz, DMSO-$d_6$) δ 10.33 (s, 2H), 8.69 (s, 2H), 7.86 (d, *J* = 8.2 Hz, 2H), 7.63 (s, 2H), 7.32 (t, *J* = 7.8 Hz, 2H), 6.99 (dd, *J* = 7.6, 1.3 Hz, 2H), 4.38 - 4.02 (m, 4H), 2.31 - 2.24 (m, 2H), 2.12 - 1.46 (m, 26H), 1.15 (d, *J* = 7.9 Hz, 4H), 0.95 - 0.78 (m, 4H).

**Example 3: preparation of 4,4'-((((((2,2'-dimethyl-[1,1'-biphenyl]-3,3'-diyl)bis(azanediyl)) bis(carbonyl))bis(4-cyclopropylpyridine-6,3-diyl))bis(methylene))bis(methylazanediyl))bis(bicyclo[2.2.1]heptane-1-carboxylic acid)**

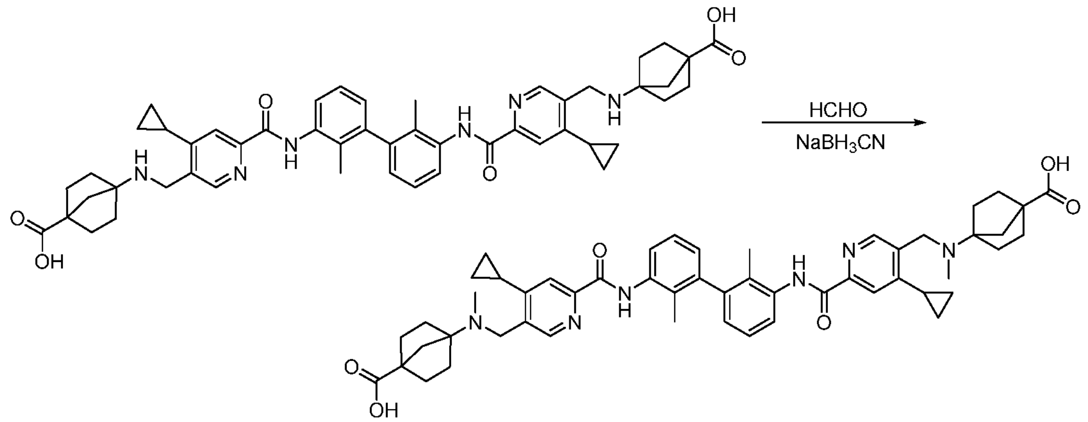


4,4'-((((((2,2'-dimethyl-[1,1'-biphenyl]-3,3'-diyl)bis(azanediyl))bis(carbonyl))bis(4-cyclopropylpyridine-6,3-diyl))bis(methylene))bis(azanediyl))bis(bicyclo[2.2.1]heptane-1-carboxylic acid) (50 mg, 0.060 mmol) was dissolved in DMF (3 mL), followed by addition of HOAc (1 mL) and aqueous formaldehyde solution (35%, 37.5 mg, 0.597 mmol). The mixture was stirred for 0.5 hrs at room temperature, and then $NaBH_3CN$ (37.5 mg, 0.597 mmol) was added. The resulting mixture was stirred overnight at room temperature under nitrogen protection. After the reaction was completed, the reaction solution was separated by reversed-phase column chromatography to obtain 4,4'-((((((2,2'-dimethyl-[1,1'-biphenyl]-3,3'-diyl)bis(azanediyl)) bis(carbonyl))bis(4-cyclopropylpyridine-6,3-diyl))bis(methylene))bis(methylazanediyl))bis(bicyclo[2.2.1]heptane-1-carboxylic acid) (12 mg, 0.013 mmol, yield: 21.6%). MS m/z (ESI): 865.6 $[M+H]^+$.

$^1$H NMR (400 MHz, DMSO-$d_6$) $\delta$ 10.30 (s, 2H), 8.52 (s, 2H), 7.90 (d, $J$ = 8.0 Hz, 2H), 7.59 (s, 2H), 7.31 (t, $J$ = 7.8 Hz, 2H), 6.97 (d, $J$ = 7.5 Hz, 2H), 3.81 (s, 4H), 2.40 (td, $J$ = 8.2, 4.2 Hz, 2H), 2.11(s, 6H), 2.05 - 1.95 (m, 10H), 1.87 - 1.57 (m, 16H), 1.12 (d, $J$ = 8.1 Hz, 4H), 0.84 (d, $J$ = 5.2 Hz, 4H).

**Example 4: preparation of 4,4'-(((((((2,2'-dimethyl-[1,1'-biphenyl]-3,3'-diyl)bis(azanediyl)) bis(carbonyl))bis(4-cyclopropylpyridine-6,3-diyl))bis(methylene))bis(azanediyl))bis(methylene))bis(bicyclo [2.2.1] heptane-1-carboxylic acid)**

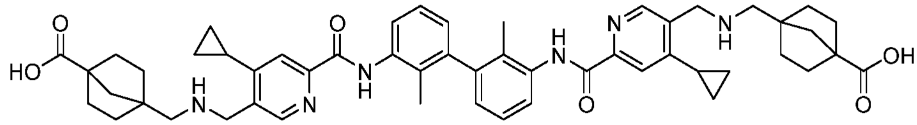

**Step 1: synthesis of methyl 4-(hydroxymethyl)bicyclo[2.2.1]heptane-1-carboxylate**

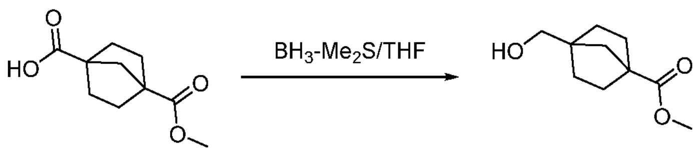


4-(methoxycarbonyl)bicyclo[2.2.1]heptane-1-carboxylic acid (800 mg, 4.04 mmol) was dissolved in dry THF (40 mL), and a $BH_3{\bullet}Me_2S$/THF solution (5.25 mL, 5.25 mmol) was slowly added dropwise under an ice-water bath. After completion of the dropwise addition, the mixture was stirred overnight at room temperature. MeOH (10 mL) was added dropwise to the reaction solution, and the mixture was refluxed for 4 hrs to quench the reaction. The reaction solution was extracted with ethyl acetate and water, and the organic phase was washed with water and saturated brine, dried over anhydrous sodium sulfate and filtered. The filtrate was concentrated, and the crude product was separated by column chromatography to obtain methyl 4-(hydroxymethyl)bicyclo[2.2.1]heptane-1-carboxylate (600 mg, yield: 80.7%).

$^1$H NMR (400 MHz, $CDCl_3$) $\delta$ 3.70 (s, 2H), 3.67 (s, 3H), 2.06 - 1.93 (m, 2H), 1.73 - 1.62 (m, 4H), 1.58 - 1.54 (m, 2H), 1.43 - 1.34 (m, 2H).

**Step 2: synthesis of methyl 4-formylbicyclo[2.2.1]heptane-1-carboxylate**

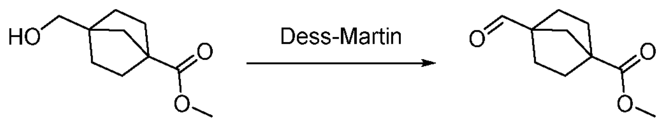


Methyl 4-(hydroxymethyl)bicyclo[2.2.1]heptane-1-carboxylate (600 mg, 3.27 mmol) was dissolved in $CH_2Cl_2$ (20 mL), followed by addition of Dess-Martin reagent (1.66 g, 3.91 mmol). The mixture was stirred at room temperature, and the reaction was monitored by TLC until the starting materials were consumed. The reaction solution was filtered through diatomaceous earth, and the filtrate was concentrated and separated by column chromatography to obtain methyl 4-formylbicyclo[2.2.1]heptane-1-carboxylate (500 mg, yield: 84.3%).

$^1H$ NMR (400 MHz, $CDCl_3$) $\delta$ 9.81 (s, 1H), 3.70 (s, 3H), 2.13 - 2.00 (m, 4H), 1.86 - 1.82 (m, 2H), 1.79 - 1.68 (m, 2H), 1.60 - 1.52 (m, 2H).

**Step 3: synthesis of methyl 4-((benzylamino)methyl)bicyclo[2.2.1]heptane-1-carboxylate**

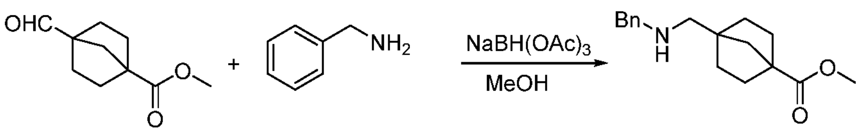


Methyl 4-formylbicyclo[2.2.1]heptane-1-carboxylate (200 mg, 1.10 mmol) and benzylamine (235 mg, 2.20 mmol) were added to a mixture solution of MeOH (20 mL) and HOAc (0.2 mL), and the mixture was stirred at room temperature for 30 min under nitrogen protection, followed by addition of $NaBH(OAc)_3$ (465 mg, 2.20 mmol). The resulting mixture was stirred overnight at room temperature. After the reaction was completed, saturated aqueous NaHCOs solution was added, and the reaction solution was extracted with $CH_2Cl_2$. The organic phase was washed with saturated brine, dried over anhydrous sodium sulfate and filtered. The filtrate was concentrated, and the crude product was separated by column chromatography to obtain methyl 4-((benzylamino)methyl)bicyclo[2.2.1]heptane-1-carboxylate (300 mg, yield: 100%). MS m/z (ESI): 274.2 $[M+H]^+$.

**Step 4: synthesis of methyl 4-(aminomethyl)bicyclo[2.2.1]heptane-1-carboxylate**

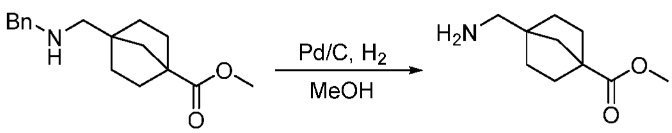


Methyl 4-((benzylamino)methyl)bicyclo[2.2.1]heptane-1-carboxylate (300 mg, 1.10 mmol) and 10% Pd/C (50 mg) were placed in methanol (30 mL), and the mixture was stirred overnight at room temperature under atmospheric hydrogen atmosphere. The reaction solution was filtered, and the filtrate was concentrated to obtain methyl 4-(aminomethyl)bicyclo[2.2.1]heptane-1-carboxylate (194 mg, yield: 96.5%). MS m/z (ESI): 184.1 $[M+H]^+$.

**Step 5: synthesis of methyl 4-({[(4-cyclopropyl-6-{[3-(3-{4-cyclopropyl-5-[({[4-(methoxycarbonyl)bicyclo[2.2.1]heptan-1-yl]methyl}amino)methyl]pyridin-2-amido}-2-methylphenyl)-2-methylphenyl]carbamoyl}pyridin-3-yl)methyl]amino}methyl)bicyclo[2.2.1]heptane-1-carboxylate**

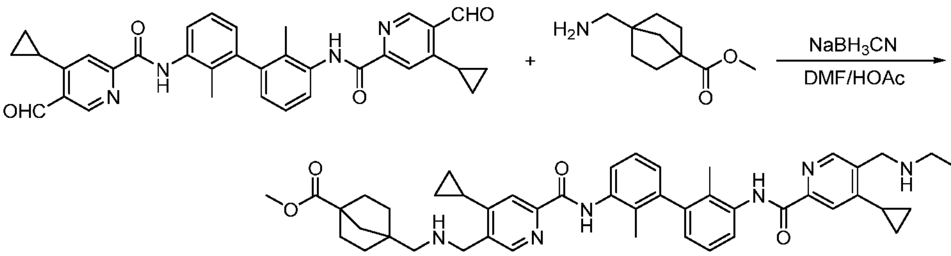


*N,N'*-(2,2'-dimethyl-[1,1'-biphenyl]-3,3'-diyl)bis(4-cyclopropyl-5-formylpicolinamide) (115 mg, 0.206 mmol) and methyl 4-(aminomethyl)bicyclo[2.2.1]heptane-1-carboxylate (189 mg, 1.03 mmol) were placed in a mixture solution of DMF (3 mL) and HOAc (0.75 mL) and the mixture was stirred at room temperature for 1 hr under nitrogen protection, followed by addition of $NaBH_3CN$ (65 mg, 1.03 mmol). The resulting mixture was stirred overnight at room temperature. After the reaction was completed, the reaction mixture was separated by reversed-phase column chromatography to obtain methyl 4-({[(4-cyclopropyl-6-{[3-(3-{4-cyclopropyl-5-[({[4-(methoxycarbonyl)bicyclo[2.2.1]heptan-1-yl]methyl} amino)methyl]pyridin-2-amido}-2-methylphenyl)-2-methylphenyl]carbamoyl}pyridin-3-yl)methyl]amino}methyl)bicyclo [2.2.1]heptane-1-carboxylate (124 mg, yield: 67.5%). MS m/z (ESI): 893.5 $[M+H]^+$.

**Step 6: synthesis of 4,4'-(((((((2,2'-dimethyl-[1,1'-biphenyl]-3,3'-diyl)bis(azanediyl)) bis(carbonyl))bis(4-cyclopropylpyridine-6,3-diyl))bis(methylene))bis(azanediyl))bis(methylene))bis(bicyclo [2.2.1] heptane-1-carboxylic acid)**

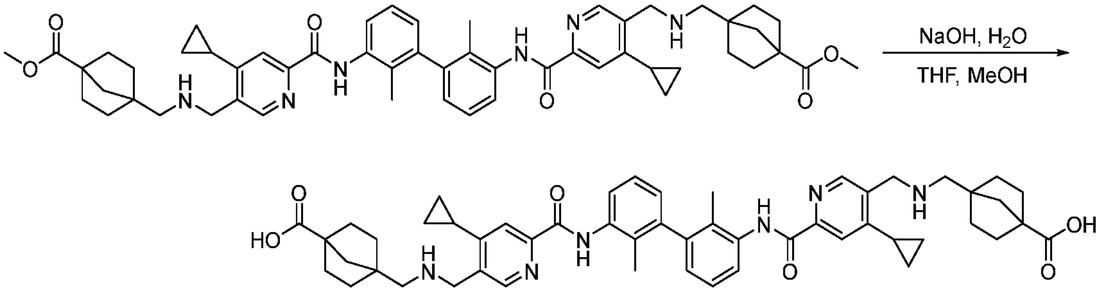


Methyl 4-({[(4-cyclopropyl-6-{[3-(3-{4-cyclopropyl-5-[({[4-(methoxycarbonyl)bicycle [2.2.1]heptan-1-yl]methyl} amino)methyl]pyridin-2-amido}-2-methylphenyl)-2-methylphenyl]carbamoyl}pyridin-3-yl)methyl]amino}methyl)bicyclo [2.2.1]heptane-1-carboxylate (124 mg, 0.139 mmol) was placed in a mixture solution of MeOH (3 mL), THF (3 mL) and water (1.5 mL), followed by addition of NaOH (83 mg, 2.08 mmol). The mixture was heated to 50 °C and stirred for 1 hr under nitrogen protection. After the reaction was completed, the reaction solution was adjusted to pH of ~6 with formic acid and then concentrated, and the crude product was separated by reversed-phase column chromatography to obtain 4,4'-(((((((2,2'-dimethyl-[1,1'-biphenyl]-3,3'-diyl)bis(azanediyl))bis(carbonyl))bis(4-cyclopropylpyridine-6,3-diyl)) bis(methylene))bis(azanediyl))bis(methylene))bis(bicyclo[2.2.1]heptane-1-carboxylic acid) formate (45.5 mg, yield: 34.1%). MS m/z (ESI): 865.5 $[M+H]^+$.

$^1H$ NMR (400 MHz, DMSO-$d_6$) δ 10.30 (s, 2H), 8.57 (s, 2H), 7.88 (d, *J* = 8.0 Hz, 2H), 7.60 (s, 2H), 7.31 (t, *J* = 7.8 Hz, 2H), 6.98 (m, 2H), 3.98 (s, 4H), 2.67 (s, 4H), 2.28 (m, 2H), 2.01 (m, 6H), 1.90 - 1.82 (m, 4H), 1.65 - 1.58 (m, 4H), 1.54 (t, *J* = 10.3 Hz, 4H), 1.45 (m, 4H), 1.32 (m, 4H), 1.15 - 1.09 (m, 4H), 0.88 - 0.82 (m, 4H).

**Example 5: preparation of 4-((((6-((3'-(5-(((4-carboxybicyclo[2.2.1]heptyl-1-yl)ethyl) aminomethyl)-4-cyclopropylmethylpyridinamido)-2,2'-dimethyl-[1,1'-biphenyl]-3-yl)carbamoyl)-4-cyclopropylpyridin-3-yl)methyl)amino) ethyl)bicyclo[2.2.1]heptyl-1-carboxylic acid**

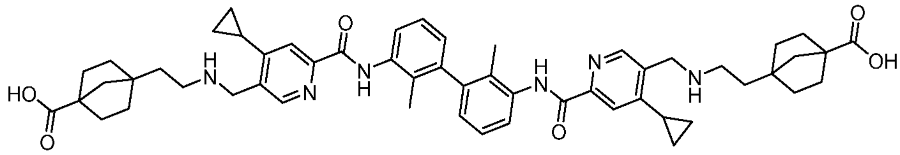

**Step 1: synthesis of methyl 4-(2-(benzylamino)ethyl)bicyclo[2.2.1]heptane-1-carboxylate**

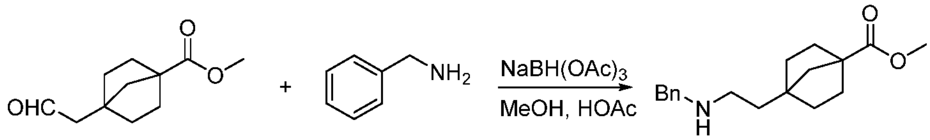


Methyl 4-(2-oxoethyl)bicyclo[2.2.1]heptane-1-carboxylate (350 mg, 1.78 mmol) and benzylamine (382 mg, 3.57 mmol) were placed in a mixture solution of MeOH (20 mL) and HOAc (0.2 mL), and the mixture was stirred at room temperature for 30 min under nitrogen protection, followed by addition of $NaBH(OAc)_3$ (756 mg, 3.57 mmol). The resulting mixture was stirred overnight at room temperature. After the reaction was completed, saturated aqueous NaHCOs solution was added, and the reaction solution was extracted with $CH_2Cl_2$. The organic phase was washed with saturated brine, dried over anhydrous sodium sulfate, and filtered. The filtrate was concentrated, and the residue was separated by column chromatography to obtain methyl 4-(2-(benzylamino)ethyl)bicyclo[2.2.1]heptane-1-carboxylate (425 mg, yield: 82.9%). MS m/z (ESI): 288.2 $[M+H]^+$.

**Step 2: synthesis of methyl 4-(2-aminoethyl)bicyclo[2.2.1]heptane-1-carboxylate**

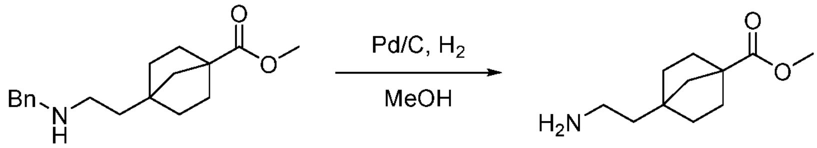


Methyl 4-(2-(benzylamino)ethyl)bicyclo[2.2.1]heptane-1-carboxylate (425 mg, 1.48 mmol) and 10% Pd/C (50 mg) were placed in methanol (30 mL), and the mixture was stirred overnight at room temperature under atmospheric hydrogen atmosphere. After the reaction was completed, the reaction solution was filtered, and the filtrate was concentrated to obtain methyl 4-(2-aminoethyl)bicyclo[2.2.1]heptane-1-carboxylate (246 mg, yield: 84.3%). MS m/z (ESI): 198.1 $[M+H]^+$.

**Step 3: synthesis of dimethyl 4,4'-(((((((2,2'-dimethyl-[1,1'-biphenyl]-3,3'-diyl)bis (azanediyl))bis(carbonyl)) bis(4-cyclopropylpyridine-6,3-diyl))bis(methylene))bis(azanediyl))bis(ethane-2,1-diyl))bis(bicyclo[2.2.1]heptane-1-carboxylate)**

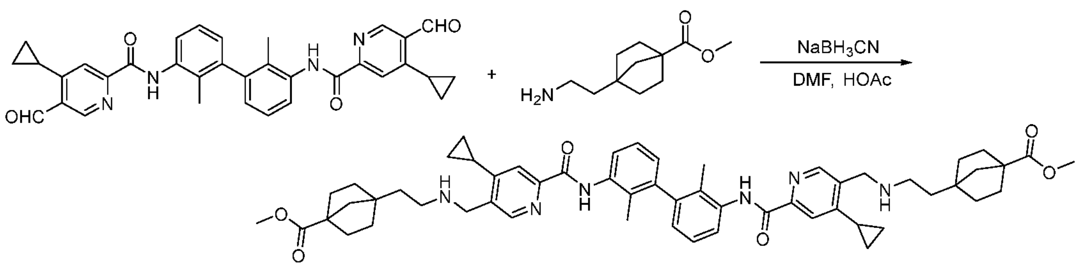


*N*,*N*-(2,2'-dimethyl-[1,1'-biphenyl]-3,3'-diyl)bis(4-cyclopropyl-5-formylpicolinamide) (110 mg, 0.197 mmol) and methyl 4-(2-aminoethyl)bicyclo[2.2.1]heptane-1-carboxylate (233 mg, 1.18 mmol) were placed in a mixture solution of

DMF (3 mL) and HOAc (0.75 mL), and the mixture was stirred at room temperature for 1 hr under nitrogen protection, followed by addition of $NaBH_3CN$ (74 mg, 1.18 mmol). The resulting mixture was stirred overnight at room temperature. After the reaction was completed, the reaction mixture was separated by reversed-phase column chromatography to obtain dimethyl 4,4'-(((((((2,2'-dimethyl-[1,1'-biphenyl]-3,3'-diyl)bis(azanediyl))bis(carbonyl))bis(4-cyclopropylpyridine-6,3-diyl))bis(methylene))bis(azanediyl))bis(ethane-2,1-diyl))bis(bicyclo[2.2.1]heptane-1-carboxylate) (100 mg, yield: 55.1%). MS m/z (ESI): 921.5 $[M+H]^+$.

**Step 4: synthesis of 4-((((6-((3'-(5-(((4-carboxybicyclo[2.2.1]heptyl-1-yl)ethyl) aminomethyl)-4-cyclopropylmethylpyridinamido)-2,2'-dimethyl-[1,1'-biphenyl]-3-yl)carbamoyl)-4-cyclopropylpyridin-3-yl)methyl)amino) ethyl)bicyclo[2.2.1]heptyl-1-carboxylic acid**

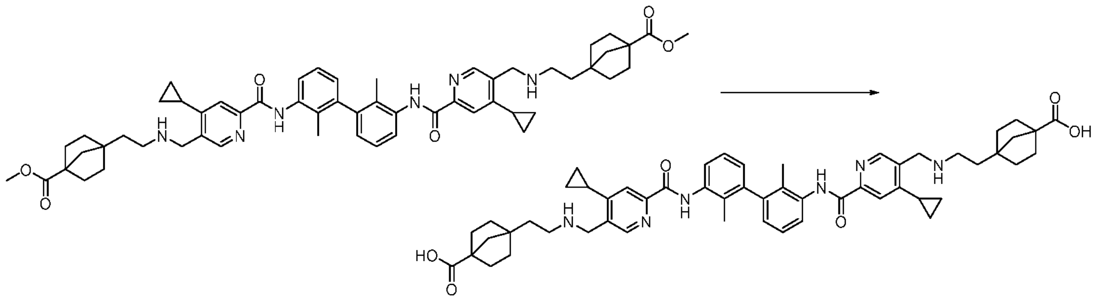

Dimethyl 4,4'-(((((((2,2'-dimethyl-[1,1'-biphenyl]-3,3'-diyl)bis(azanediyl))bis(carbonyl)) bis(4-cyclopropylpyridine-6,3-diyl))bis(methylene))bis(azanediyl))bis(ethane-2,1-diyl))bis(bicyclo[2.2.1]heptane-1-carboxylate) (100 mg, 0.108 mmol) was placed in a mixture solution of methanol (3 mL), THF (3 mL) and water (1.5 mL), followed by addition of NaOH (90 mg, 2.25 mmol). The mixture was heated to 50 °C and stirred for 1 hr. After the reaction was completed, the reaction solution was adjusted to pH of ~6 with formic acid and then concentrated, and the residue was separated by reversed-phase column chromatography to obtain 4-((((6-((3'-(5-(((4-carboxybicyclo[2.2.1]heptyl-1-yl)ethyl)aminomethyl)-4-cyclopropylmethylpyridinamido)-2,2'-dimethyl-[1,1'-biphenyl]-3-yl)carbamoyl)-4-cyclopropylpyridin-3-yl) methyl)amino)ethyl)bicyclo[2.2.1]heptyl-1-carboxylic acid (45.5 mg, yield: 34.1%). MS m/z (ESI): 893.5 $[M+H]^+$.

$^1$H NMR (400 MHz, DMSO-$d_6$) δ 10.30 (s, 2H), 8.55 (s, 2H), 7.88 (m, 2H), 7.58 (s, 2H), 7.31 (t, $J$ = 7.8 Hz, 2H), 6.98 (m, 2H), 3.96 (s, 4H), 2.63 (t, $J$ = 7.8 Hz, 4H), 2.29 (m, 2H), 2.00(s, 6H), 1.86 (m, 4H), 1.69 (t, $J$ = 7.8 Hz, 4H), 1.56 - 1.30 (m, 16H), 1.16 - 1.10 (m, 4H), 0.87 - 0.82 (m, 4H).

## Biological Test Evaluation

### 1. PD1-PDL1 HTRF binding activity test

The effect of compounds of the examples of the present invention on the PD-1/PD-L1 interaction was determined by the PD-1/PD-L1 binding assay kit from Cisbio (#64ICP01PEG or 64ICP01PEH). The detailed experimental process was as follows:

1) pre-diluted compound solution, 4 μL of Tag1-PD-L1 and 4 μL of Tag2-PD1 were each added to a 384-well plate;

2) after the mixture was incubated at room temperature for 15 min, 5 μL of anti-Tag1-Eu3+ antibody and 5 μL of anti-Tag2-XL665 antibody were then added;

3) after being incubated for 2 hrs at room temperature or overnight at 4 °C, the plate was read on Envision of Perkin Elmer; readings at 665 nm and 620 nm were recorded, and the ratio of the two readings was taken as the reading of each well;

4) the reading of each well after compound treatment was compared with the reading of DMSO treated wells to obtain the percent inhibition of the compound; and

5) $IC_{50}$ values of compounds of the examples of the present invention were determined by non-linear regression analysis of percent inhibition at different compound concentrations. The specific experimental results are shown in

Table 1.

**2. Jurkat reporter gene cellular assay**

The effect of compounds of the examples of the present invention on the PD-1/PD-L1 interaction expressed on cell surfaces and the related influence on T cell functions were determined by a Jurkat reporter gene cellular assay. Briefly, the reporter gene plasmid of NF-κB-luc and the plasmid of human PD-1 were transfected into Jurkat cells to establish a stably transfected cell line capable of stably expressing both PD-1 and NF-xB-Luc reporter genes; the expression level of PD-1 on the cell surface was confirmed by flow cytometry; and the expression of the reporter gene was confirmed via the response of the reporter gene stimulated by OKT-3 and Raji cells.

In addition, the plasmid of human PD-L1 was transfected into Raji cells to obtain a cell line capable of stably expressing PD-L1. Jurkat/NF-κB-luc/PD1 cells and Raji-PD-L1 cells were then cocultured and stimulated with OKT-3. On this basis, the compounds were added, and the enhancement of the signal pathway of T cell activation by the inhibitory effect of the compounds on the PD-1/PD-L1 interaction was evaluated by readings of reporter gene responses. The specific experimental process was as follows:

1) 30 μL of compound or antibody solution at different diluted concentrations was added to a white 96-well plate (Corning, 3610), and 10 μL of OKT3 (Biolegend, 317326) was then added (the final concentration of OKT3: 1 μg/mL);

2) 20 μL of Raji-PD-L1 cell suspension was added to each well with 5 * $10^4$ cells for each well, and the mixture was incubated in an incubator for 20 min;

3) 20 μL of Jurkat/NF-κb-luc/PD-1 cell suspension was added to each well with 5 * $10^4$ cells for each well and well mixed, and 6 hrs later, Bright-glo (Promega, E2620) was applied;

4) the reading of each well after compound treatment was compared with the reading of DMSO treated wells to obtain the activation fold of the compound; and

5) $EC_{50}$ values of compounds of the examples of the present invention were determined by non-linear regression analysis of activation folds at different compound concentrations. The specific experimental results are shown in Table 1:

**Table 1: Biological test results**

| Example No. | PD1-PDL1 HTRF binding activity $IC_{50}$/nM | Cell activity $EC_{50}$/nM |
|---|---|---|
| **1** | 2.56 | 135 |
| **2** | 3.67 | 151 |
| **3** | 4.62 | 1383 |
| **4** | 1.46 | 247 |
| **5** | 4.19 | 163 |

The bioactivity data of the compounds of the specific examples indicate that the series of compounds of the present invention have a strong inhibitory effect on the PD-1/PD-L1 interaction, and moreover, such an inhibitory effect can enhance or recover the activation of T cells at the cellular level.

**3. Pharmacokinetic evaluation (evaluation of the membrane permeability and intestinal absorption potential of candidate drugs by Caco-2 cell line)**

In this experiment, Caco-2 cells adopted were human colon cancer cells. Similar to normal intestinal epithelium, the Caco-2 cells were able to form differentiated monolayer cells with a plurality of characteristics under proper culture conditions, such as tight connection, expression of microvilli and brush border enzyme, and the like. The permeability of compounds on a Caco-2 cell model is closely related to the absorption in a human body, and the Caco-2 cell model is widely applied to the evaluation of the permeability and the active transport process of compounds in the intestinal tract. The permeability and the efflux ratio of the compounds of the examples of the present invention and the comparative compounds were evaluated using a Caco-2 cell model in this experiment. The specific process was as follows:

1) The Caco-2 cell culture medium was modified Eagle's medium (MEM) containing 10% inactivated fetal bovine serum and 1% nonessential amino acids. Cells were plated on the polycarbonate filter membrane and cultured in an incubator at 37 °C/5% $CO_2$.

2) The cells could be used for a transport experiment after being cultured for 21-28 days following plating, and the compactness of a cell monolayer was characterized and verified through the Lucifer Yellow apparent permeability coefficient ($P_{app}$).

3) In the experiment, 10 mM stock solutions were prepared by dissolving the compounds in DMSO, and reference

compounds used in this experiment for evaluating permeability were metoprolol, atenolol and erythromycin, which were used as positive compounds for high permeability and low permeability and positive substrate for P-gp, respectively. The apparent permeability coefficients determined for the reference compounds obtained according to the experimental process are as follows:

| **Reference compound** | **$P_{app}$ ($10^{-6}$ cm·s$^{-1}$)** | | **Papp(B-A)/ Papp(A-B)** | Evaluation of permeability |
|---|---|---|---|---|
| | A-B | B-A | | |
| **Metoprolol** | 24.70 | 31.13 | 1.26 | High permeability |
| **Atenolol** | 0.42 | 0.57 | 1.37 | Low permeability |
| **Erythromycin** | 0.20 | 21.65 | 107.3 | High efflux ratio |

Working solutions were obtained by diluting with Hank's balanced salt solution (HBSS, Invitrogen) containing 25 mM HEPES (pH 7.4). The detection concentration of the test compound was 10 μM. This study was a two-way permeation experiment from the apical side to the basolateral side (A-B) and from the basolateral side to the apical side (B-A), and a 90-minute incubation at 37 °C was required. After the incubation, the samples diluted with buffer were detected by LC-MS/MS. The concentrations of the compounds were quantified using a standard curve.

4) Apparent permeability coefficient $P_{app}$ value (cm/s):

$$P_{app} = (VA / (Area \times time)) * ([drug]accepter /(([drug]initial, donor) * Dilution Factor)$$

Wherein, "VA" refers to the volume in the acceptor pore, "Area" refers to the surface area of the membrane, and "time" is the total transport time in seconds.

5) The apparent membrane permeability coefficient value $P_{app}$ and the efflux ratio ER of the respective compounds were determined according to the above calculation formula and the formula ER = $P_{app}$(B-A)/ $P_{app}$(A-B), and the results are shown in Table 2:

**Table 2. Results of Caco-2 membrane permeability test**

| **Example No.** | **$P_{app}$ ($10^{-6}$ cm·s$^{-1}$)** | | **$P_{app}$(B-A)/ $P_{app}$(A-B)** |
|---|---|---|---|
| | A-B | B-A | |
| **1** | 2.46 | 16.53 | 6.75 |
| **4** | 9.79 | 21.68 | 2.21 |
| **5** | 2.45 | 15.68 | 6.40 |
| **Comparative compound 1** | <2.28* | <1.85* | / |
| **Comparative compound 2** | 0.67 | 0.36 | 0.53 |
| **Comparative compound 3** | N/A | N/A | / |
| **Comparative compound 4** | <0.24* | 0.98 | / |
| **Comparative compound 5** | <1.79 | <0.65 | / |
| **Comparative compound 6** | 0.34 | 5.51 | 16.26 |
| *if **$P_{app}$ values are expressed as** "<" a value, it means that the value is calculated using the minimum standard concentration at the receiver side, which depends on the limit of quantitation of the true concentration at the receiver side. **"N/A"** means not detectable. | | | |

**Table 3. Structures of comparative compounds**

| Comparative compound | | Structural formula |
|---|---|---|
| 1 | WO2019149183<br>Example 104 | 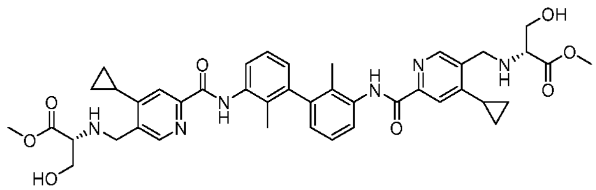 |
| 2 | WO2019149183<br>Example 105 | 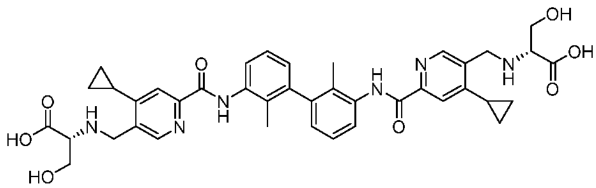 |
| 3 | WO2019149183<br>Example 121 | 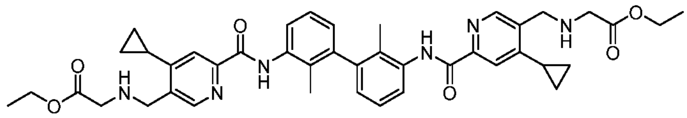 |
| 4 | WO2019149183<br>Example 122 | 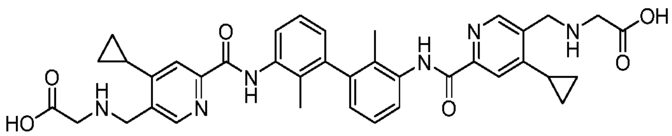 |
| 5 | WO2019149183<br>Example 133 | 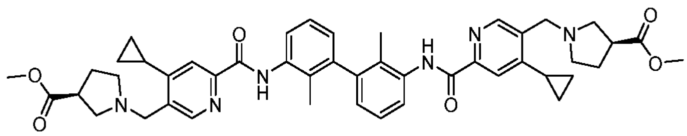 |
| 6 | WO2019149183<br>Example 134 | 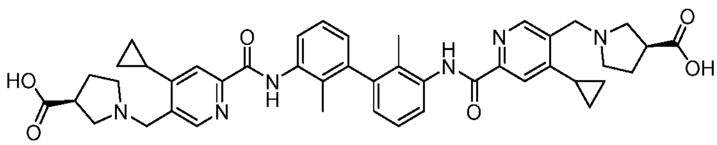 |

The data of the membrane permeability test in Table 2 show that the compounds of the examples of the present invention have significantly improved A-B membrane permeability due to modification with the linker groups at the two ends of the compounds, while the comparative compounds 2, 4, and 6 all have low A-B $P_{app}$ value and poor membrane permeability although they also have a symmetric diacid structure. In addition, through *in vivo* pharmacokinetic experiments of mice, the inventors further found that these compounds were hardly absorbed if directly administered orally and oral administration was possible only by administering their prodrugs (comparative compounds 1, 3 and 5), and these compounds were different from the compounds of the examples of the present invention in metabolic pathway.

**4. Evaluation of pharmacokinetics in mice and rats**

**1. Purpose of study**

The purpose of this study is to study the pharmacokinetic behaviors of some compounds of the present invention, and administration routes were: single per oral administration (PO) or intravenous injection (IV) to ICR mice or rats.

**2. Test scheme**

**2.1 Test drugs**

The compounds used in this test were compounds of specific examples of the present invention and the comparative compounds listed.

### 2.2 Test animals

ICR mice male N = 9 original source: Shanghai Sippr-BK Laboratory Animal Co. Ltd.
ICR rats male N = 3 original source: Shanghai Sippr-BK Laboratory Animal Co. Ltd.

### 2.3 Preparation and administration of drugs

The compounds were each dissolved in a corresponding vehicle, mixed well by shaking and subjected to ultrasonic treatment to obtain colorless clear solutions. 9 mice and 3 rats were orally administered with the solution after fasting overnight. The mice were dosed at 10 mg/kg (or 2 mg/kg by IV) and the rats were dosed at 5 mg/kg (or 1 mg/kg by IV).

### 2.4 Sampling

With about 90 μL/time point, blood was drawn from the submaxillary vein, and heparin sodium was added for anticoagulation. The blood samples were placed onto the ice and centrifuged (centrifugation conditions: 8000 rpm, 6 min, 2-8 °C) within 1 hr to isolate the plasmas. The time points for blood sampling were at 0 hrs, 0.25 hrs, 0.5 hrs, 1 hr, 2 hrs, 4 hrs, 6 hrs, 8 hrs and 24 hrs after administration. The samples were stored in a refrigerator at -20 °C.
40 μL of plasma sample was added to 160 μL of cold acetonitrile containing an internal standard, and the mixture was vortexed for 3 min and centrifuged at 11,000 rpm for 5 min. 100 μL of the supernate was added to 100 μL of water, and 5 μL of the mixed solution was loaded for LC-MS/MS analysis. For comparative compounds 1, 3, 5, the prodrug compounds and corresponding prototype compounds 2, 4, 6 were all analyzed, and specific analysis results are shown in Table 4.

### 2.5 Test results

**Table 4. Exposure and oral availability of test compounds in mice**

| Example No. | Administration dose | $C_{max}$ (ng/mL) | $AUC_{last}$ (hr*ng/mL) | Oral availability (F%) |
|---|---|---|---|---|
| **Example 1** | PO (10 mpk) | 1912.1 | 8786.2 | 19.5% |
| | IV (2 mpk) | 4003.3 | 8995.8 | / |
| **Example 4** | PO (10 mpk) | 4546.7 | 17564.0 | 57.5% |
| | IV (2 mpk) | 3523.3 | 6109.2 | / |
| **Example 5** | PO (10 mpk) | 2983.3 | 14569.9 | 134% |
| | IV (2 mpk) | 1040.3 | 2169.4 | / |
| **Comparative compound 1** | PO (10 mpk) | 0 | 0 | 0% |
| | Detection of comparative compound 2 | 1813.3 | 3968 | 33.7% |
| **Comparative compound 2** | PO (10 mpk) | 0 | 0 | 0% |
| | IV (2 mpk) | 5640.0 | 2355 | / |
| **Comparative compound 3** | PO (10 mpk) | 0 | 0 | 0% |
| | Detection of comparative compound 4 | 412.3 | 592 | / |
| **Comparative compound 5** | PO (10 mpk) | 30.4 | / | / |
| | Detection of comparative compound 6 | 437.7 | 866 | / |
| **Comparative compound 6** | PO (10 mpk) | 46.2 | 147 | / |

Experiments show that compared with comparative compounds, the compounds of the examples of the present invention have significantly improved oral exposure and good oral availability and are sufficient to support the development of oral administration agents in the next step.

**Table 5. Exposure and oral availability of test compounds in rats**

| Example No. | Administration dose | | $C_{max}$ (ng/mL) | $AUC_{last}$ (hr*ng/mL) | Oral availability (F%) |
|---|---|---|---|---|---|
| Example 1 | PO | (5 mpk) | 426.0 | 2222.6 | 18.9% |
| | IV | (1 mpk) | 3537 | 2351 | / |
| Example 4 | PO | (5 mpk) | 1980 | 4836 | 44% |
| | IV | (1 mpk) | 1863 | 2197 | / |
| Example 5 | PO | (5 mpk) | 492 | 1607 | 15.9% |
| | IV | (1 mpk) | 1883 | 2023 | / |
| Comparative compound 1 | PO | (5 mpk) | 0 | 0 | 0% |
| | Detection of comparative compound 2 | | 266 | 629 | 1.6% |
| Comparative compound 2 | PO | | / | / | / |
| | IV | (1 mpk) | 13167 | 7642 | / |

Experiments show that compared with comparative compounds, the compounds of the examples of the present invention have significantly improved oral exposure and good oral availability and are sufficient to support the development of oral administration agents in the next step.

All documents mentioned in the present invention are incorporated as references, just as each document is individually cited as a reference. In addition, it should be understood that various modifications or changes may be made by those skilled in the art after reading the above disclosure of the present invention, and these equivalent forms also fall within the scope defined by the claims appended hereto.

## Claims

**1.** A compound of formula (I), a stereoisomer or pharmaceutically acceptable salt thereof:

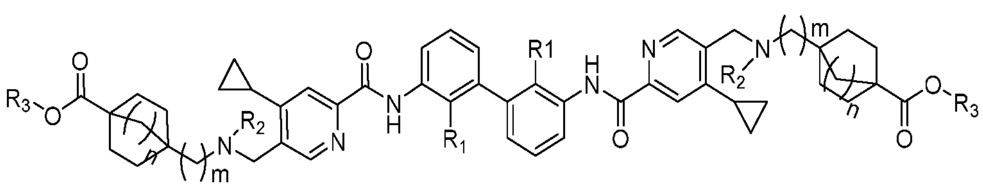

**(I)**

wherein, both $R_1$ are identical or different and are each independently selected from the group consisting of hydrogen, deuterium, halogen, cyano, $C_{1-4}$ alkyl and $C_{3-6}$ cycloalkyl, the $C_{1-4}$ alkyl and $C_{3-6}$ cycloalkyl are further optionally substituted by one or more substituents selected from the group consisting of deuterium, halogen, cyano, $C_{1-4}$ alkyl, $C_{1-4}$ haloalkyl, $C_{1-4}$ deuterioalkyl, cyclopropyl, hydroxy and $C_{1-4}$ alkoxy;

both $R_2$ are identical or different and are each independently selected from the group consisting of hydrogen, deuterium, $C_{1-4}$ alkyl, $C_{3-6}$ cycloalkyl and hydroxy, the $C_{1-4}$ alkyl and $C_{3-6}$ cycloalkyl are further optionally substituted by one or more substituents selected from the group consisting of deuterium, halogen, cyano, $C_{1-4}$ alkyl, $C_{1-4}$ haloalkyl, $C_{1-4}$ deuterioalkyl, cyclopropyl, hydroxy and $C_{1-4}$ alkoxy;

both $R_3$ are identical or different and are each independently selected from the group consisting of hydrogen, deuterium, $C_{1-10}$ alkyl, $C_{3-10}$ cycloalkyl and 3-10 membered heterocyclyl, the $C_{1-10}$ alkyl, $C_{3-10}$ cycloalkyl and 3-10 membered heterocyclyl are further optionally substituted by one or more substituents selected from the group consisting of deuterium, halogen, cyano, hydroxy, carboxyl, $C_{1-4}$ alkyl, $C_{1-4}$ haloalkyl, $C_{1-4}$ deuterioalkyl, $C_{3-6}$ cycloalkyl, 3-6 membered heterocyclyl, $C_{6-10}$ aryl, 5-8 membered heteroaryl, $C_{3-6}$ cycloalkoxy, 3-6 mem-

bered heterocycloxy and $C_{1-4}$ alkoxy;
both m are identical or different and are each independently 0, 1, 2, 3 or 4;
both n are identical or different and are each independently 0, 1, 2, 3 or 4.

**2.** The compound of formula (I), the stereoisomer or pharmaceutically acceptable salt thereof of claim 1, wherein both $R_1$ are identical or different and are each independently selected from the group consisting of hydrogen, deuterium, halogen, cyano, $C_{1-4}$ alkyl, $C_{1-4}$ haloalkyl, $C_{1-4}$ deuterioalkyl, cyclopropyl-substituted $C_{1-4}$ alkyl and $C_{3-6}$ cycloalkyl; **preferably,** both $R_1$ are identical or different and are each independently selected from the group consisting of hydrogen, deuterium, fluoro, chloro, cyano, methyl, ethyl, isopropyl, trifluoromethyl, trideuteriomethyl, cyclopropylmethyl and cyclopropyl.

**3.** The compound of formula (I), the stereoisomer or pharmaceutically acceptable salt thereof of claim 1, wherein both $R_2$ are identical or different and are each independently selected from the group consisting of hydrogen, deuterium, $C_{1-4}$ alkyl, $C_{1-4}$ haloalkyl, $C_{1-4}$ deuterioalkyl, cyclopropyl-substituted $C_{1-4}$ alkyl, $C_{3-6}$ cycloalkyl and hydroxy; **preferably,** both $R_2$ are identical or different and are each independently selected from the group consisting of hydrogen, deuterium, methyl, ethyl, isopropyl, trifluoromethyl, trideuteriomethyl, cyclopropylmethyl, cyclopropyl and hydroxy.

**4.** The compound of formula (I), the stereoisomer or pharmaceutically acceptable salt thereof of claim 1, wherein both $R_3$ are identical or different and are each independently selected from the group consisting of hydrogen, deuterium, $C_{1-4}$ alkyl, $C_{3-6}$ cycloalkyl and 3-6 membered heterocyclyl, the $C_{1-4}$ alkyl, $C_{3-6}$ cycloalkyl and 3-6 membered heterocyclyl are further optionally substituted by one or more substituents selected from the group consisting of deuterium, halogen, cyano, hydroxy, carboxyl, $C_{1-4}$ alkyl, $C_{1-4}$ haloalkyl, $C_{1-4}$ deuterioalkyl, $C_{3-6}$ cycloalkyl, 3-6 membered heterocyclyl, $C_{3-6}$ cycloalkoxy and $C_{1-4}$ alkoxy;

**preferably,** both $R_3$ are identical or different and are each independently selected from the group consisting of hydrogen, deuterium, methyl, ethyl, isopropyl, cyclopropyl, cyclobutyl, cyclopentyl, oxacyclobutyl and azacyclobutyl, the methyl, ethyl, isopropyl, cyclopropyl, cyclobutyl, oxacyclobutyl and azacyclobutyl are further optionally substituted by one or more substituents selected from the group consisting of deuterium, halogen, cyano, hydroxy, carboxyl, methyl, ethyl, isopropyl, trifluoromethyl, trideuteriomethyl, cyclopropyl, cyclobutyl, oxacyclobutyl, azacyclobutyl, cyclopropyloxy, cyclobutyloxy, methoxy, ethoxy and isopropoxy;
**more preferably,** both $R_3$ are identical or different and are each independently selected from the group consisting of hydrogen, deuterium, methyl, ethyl, isopropyl, trifluoromethyl, trideuteriomethyl, cyclopropylmethyl, cyclopropyl, cyclobutyl, oxacyclobutyl and azacyclobutyl.

**5.** The compound of formula (I), the stereoisomer or pharmaceutically acceptable salt thereof of claim 1, wherein both $R_1$ are identical and are selected from the group consisting of hydrogen, deuterium, fluoro, chloro, cyano, methyl, ethyl, isopropyl, trifluoromethyl, trideuteriomethyl, cyclopropylmethyl and cyclopropyl;

both $R_2$ are identical and are selected from the group consisting of hydrogen, deuterium, methyl, ethyl, isopropyl, trifluoromethyl, trideuteriomethyl, cyclopropylmethyl, cyclopropyl and hydroxy;
both $R_3$ are identical and are selected from the group consisting of hydrogen, deuterium, methyl, ethyl, isopropyl, trifluoromethyl, trideuteriomethyl, cyclopropylmethyl, cyclopropyl, cyclobutyl, oxacyclobutyl and azacyclobutyl;
both m are identical and are 0, 1 or 2;
both n are identical and are 1 or 2.

**6.** The compound of formula (I), the stereoisomer or pharmaceutically acceptable salt thereof of claim 1, wherein the compound is selected from the group consisting of the following compounds:

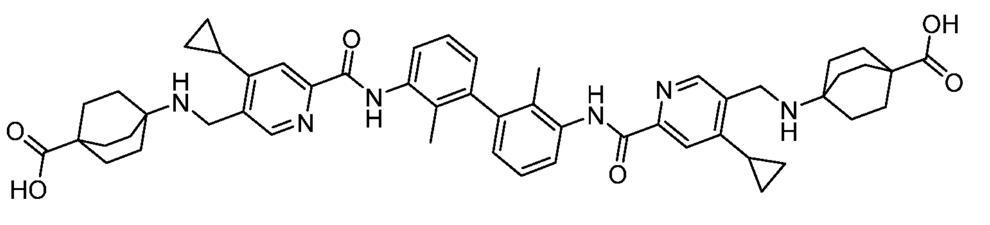

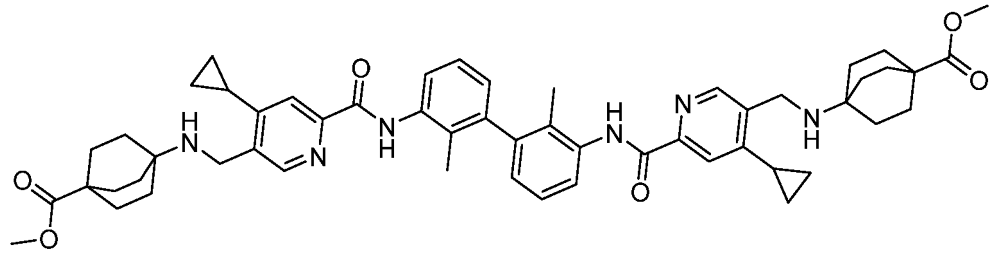

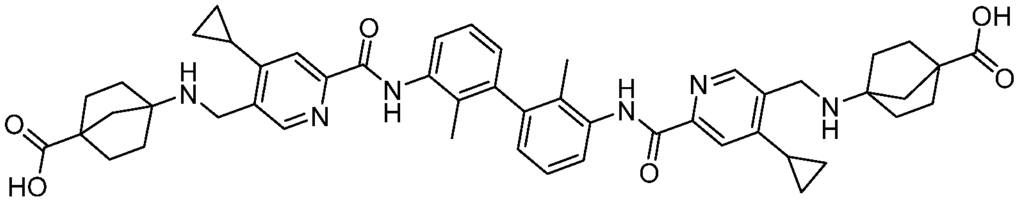

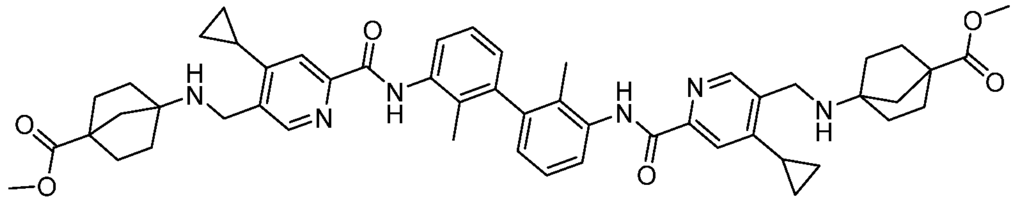

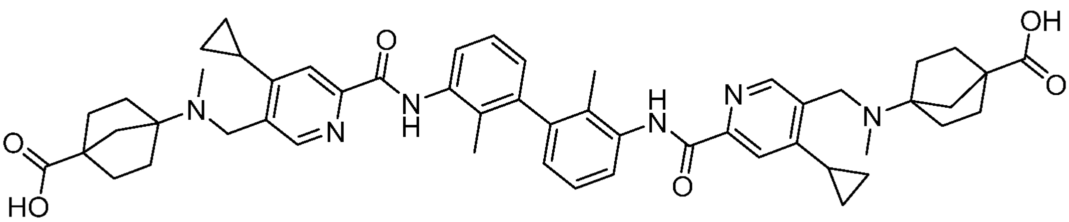

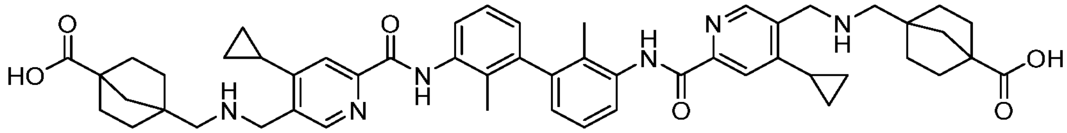

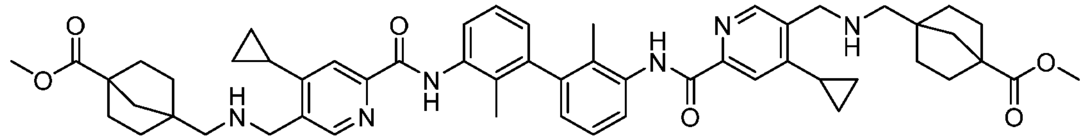

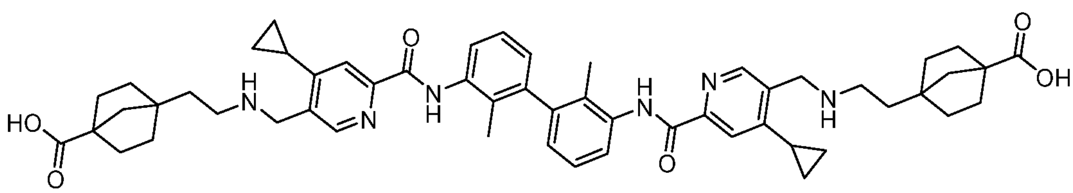

and

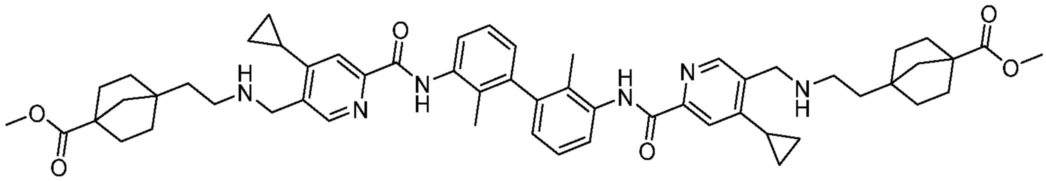

.

7. A pharmaceutical composition, comprising the compound of formula (I), the stereoisomer or pharmaceutically acceptable salt thereof of any one of claims 1-6 and a pharmaceutically acceptable carrier.

8. Use of the compound of formula (I), the stereoisomer or pharmaceutically acceptable salt thereof of any one of claims 1-6 in the preparation of a medicament for treating a PD-1/PD-L1 signal pathway-mediated disease.

9. The use of claim 8, wherein the PD-1/PD-L1 signal pathway-mediated disease is cancer or tumor, immune-related disease and disorder, communicable disease, infectious disease or metabolic disease.

10. The use of claim 9, wherein the cancer or tumor is lymphoma (including but not limited to lymphocytic lymphoma, primary central nervous system lymphoma, T cell lymphoma, diffuse large B cell lymphoma, follicle center lymphoma, Hodgkin lymphoma, non-Hodgkin lymphoma or primary mediastinal large B cell lymphoma), sarcoma (including but not limited to Kaposi's sarcoma, fibrosarcoma, liposarcoma, chondrosarcoma, osteosarcoma, leiomyosarcoma, rhabdomyosarcoma, soft tissue sarcoma, angiosarcoma or lymphangiosarcoma), melanoma, glioblastoma, synovioma, meningioma, biliary tract tumor, thymic tumor, neuroma, seminoma, nephroblastoma, pleomorphic adenoma, hepatocellular papilloma, renal tubule adenoma, cystadenoma, papilloma, adenoma, leiomyoma, rhabdomyoma, hemangioma, lymphangioma, osteoma, chondroma, lipoma, fibroma, central nervous system tumor, rhachiophyma, brain stem glioma, pituitary adenoma, multiple myeloma, ovarian tumor, myelodysplastic syndrome or mesothelioma, prostate cancer, recurrent prostate cancer or prostate cancer having developed resistance to existing medicaments, thyroid cancer, parathyroid cancer, anal cancer, testicular cancer, urethral carcinoma, penile cancer, bladder cancer, ureteral cancer, uterine cancer, ovarian cancer, fallopian tube cancer, endometrial cancer, cervical cancer, vaginal cancer, vulvar cancer, adrenal cancer, Merkel cell carcinoma, embryonal carcinoma, chronic or acute leukemia (including but not limited to acute myeloid leukemia, chronic myeloid leukemia, acute lymphoblastic leukemia, chronic granulocytic leukemia and chronic lymphoblastic leukemia), bronchial carcinoma, esophageal cancer, nasopharyngeal carcinoma, hepatocellular carcinoma, renal cell carcinoma, small cell lung cancer, basal cell carcinoma, lung cancer, breast cancer, adenocarcinoma, papillary carcinoma, cystadenocarcinoma, squamous non-small cell lung cancer, non-squamous non-small cell lung cancer, rectal cancer, colon cancer, colorectal cancer, gastric cancer, pancreatic cancer, head and neck squamous cell carcinoma, head and neck cancer, gastrointestinal cancer, bone cancer, skin cancer, small intestine cancer, endocrine cancer, renal pelvic carcinoma, epidermoid carcinoma, abdominal wall carcinoma, renal cell carcinoma, transitional cell carcinoma, choriocarcinoma or metastatic tumor;

the immune-related disease and disorder is rheumatic arthritis, renal failure, lupus erythematosus, asthma, psoriasis, ulcerative colitis, pancreatitis, allergy, fibrosis, anemia, fibromyalgia, Alzheimer's disease, congestive heart failure, stroke, aortic valve stenosis, arteriosclerosis, osteoporosis, Parkinson's disease, infection, Crohn's disease, ulcerative colitis, allergic contact dermatitis and eczema, systemic sclerosis or multiple sclerosis;
the infectious disease is bacterial infectious disease, viral infectious disease or fungal infectious disease;
the communicable disease or infectious disease is sepsis, liver infection, HIV, hepatitis A, hepatitis B, hepatitis C, hepatitis D, herpes virus, papillomavirus or influenza;
the metabolic disease is diabetes, diabetic ketoacidosis, hyperglycemic hyperosmolar syndrome, hypoglycemia, gout, malnutrition, vitamin A deficiency, scurvy, vitamin D deficiency or osteoporosis.

11. The compound of formula (I), the stereoisomer or pharmaceutically acceptable salt thereof of any one of claims 1-6 for use as a medicament for treating PD-1/PD-L1 signal pathway-mediated cancer or tumor, immune-related disease and disorder, communicable disease, infectious disease or metabolic disease.

**INTERNATIONAL SEARCH REPORT**

International application No.
**PCT/CN2023/079912**

**A. CLASSIFICATION OF SUBJECT MATTER**

C07D213/81(2006.01)i;C07D401/12(2006.01)i;C07D401/02(2006.01)i;A61K31/443(2006.01)i;A61K31/4433(2006.01)i;A61K31/4439(2006.01)i;A61K31/444(2006.01)i;A61P35/00(2006.01)i;A61P37/02(2006.01)i

According to International Patent Classification (IPC) or to both national classification and IPC

**B. FIELDS SEARCHED**

Minimum documentation searched (classification system followed by classification symbols)

IPC： C07D A61K A61P

Documentation searched other than minimum documentation to the extent that such documents are included in the fields searched

Electronic data base consulted during the international search (name of data base and, where practicable, search terms used)

CNABS; CNTXT; VEN; WOTXT; EPTXT; USTXT; STN; CNKI; ISI web of science: 上海和誉生物医药科技有限公司, 杨飞, 张永贤, 喻红平, 陈椎, 徐耀昌, 联苯基, 联芳基, PD-1, PD-L1, 癌症, 肿瘤, 免疫, biphenyl, biaryl, cancer, tumor, tumour, immun+, structural formula (I)

**C. DOCUMENTS CONSIDERED TO BE RELEVANT**

| Category* | Citation of document, with indication, where appropriate, of the relevant passages | Relevant to claim No. |
|---|---|---|
| X | WO 2019149183 A1 (SHANGHAI ABBISKO THERAPEUTICS CO., LTD.) 08 August 2019 (2019-08-08)<br>claims 1-22 | 1-4, 7-11 |
| A | WO 2019149183 A1 (SHANGHAI ABBISKO THERAPEUTICS CO., LTD.) 08 August 2019 (2019-08-08)<br>claims 1-22 | 5, 6 |
| A | WO 2021254005 A1 (SHANGHAI ABBISKO THERAPEUTICS CO., LTD.) 23 December 2021 (2021-12-23)<br>entire document | 1-11 |
| A | WO 2021008491 A1 (SHANGHAI ABBISKO THERAPEUTICS CO., LTD.) 21 January 2021 (2021-01-21)<br>entire document | 1-11 |
| A | CN 111936475 A (BETTA PHARMACEUTICALS CO., LTD.) 13 November 2020 (2020-11-13)<br>entire document | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 April 2023** | **01 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/079912**

**C. DOCUMENTS CONSIDERED TO BE RELEVANT**

| Category* | Citation of document, with indication, where appropriate, of the relevant passages | Relevant to claim No. |
|---|---|---|
| A | WO 2019191624 A1 (ARBUTUS BIOPHARMA, INC.) 03 October 2019 (2019-10-03) entire document | 1-11 |
| A | CN 113801111 A (SHANGHAI HANSOH BIOMEDICAL CO., LTD. et al.) 17 December 2021 (2021-12-17) entire document | 1-11 |

Form PCT/ISA/210 (second sheet) (July 2022)

**INTERNATIONAL SEARCH REPORT**
**Information on patent family members**

International application No.
**PCT/CN2023/079912**

| Patent document cited in search report | Publication date (day/month/year) | Patent family member(s) | Publication date (day/month/year) |
|---|---|---|---|
| WO 2019149183 A1 | 08 August 2019 | CA 3088927 A1 | 08 August 2019 |
| | | TW 201934544 A | 01 September 2019 |
| | | AU 2019214089 A1 | 09 July 2020 |
| | | KR 20200100717 A | 26 August 2020 |
| | | SG 11202006409 A1 | 28 August 2020 |
| | | HK 40023971 A0 | 04 December 2020 |
| | | BR 112020014459 A2 | 15 December 2020 |
| | | EP 3750887 A1 | 16 December 2020 |
| | | US 2021032270 A1 | 04 February 2021 |
| | | AU 2019214089 B2 | 11 February 2021 |
| | | PH 12020551184 A1 | 10 May 2021 |
| | | JP 2021512878 A | 20 May 2021 |
| | | EP 3750887 A4 | 20 October 2021 |
| | | JP 7033343 B2 | 10 March 2022 |
| | | RU 2020127950 A | 11 March 2022 |
| | | RU 2768830 C2 | 24 March 2022 |
| | | RU 2768830 C9 | 17 June 2022 |
| | | US 11459339 B2 | 04 October 2022 |
| | | MX 398873 B | 04 January 2023 |
| WO 2021254005 A1 | 23 December 2021 | TW 202200575 A | 01 January 2022 |
| | | TW 781607 B1 | 21 October 2022 |
| | | CN 115605475 A | 13 January 2023 |
| | | CA 3182595 A1 | 23 December 2021 |
| WO 2021008491 A1 | 21 January 2021 | CN 113825751 A | 21 December 2021 |
| | | EP 4001274 A1 | 25 May 2022 |
| | | US 2022380342 A1 | 01 December 2022 |
| CN 111936475 A | 13 November 2020 | WO 2019192506 A1 | 10 October 2019 |
| | | KR 20210010979 A | 29 January 2021 |
| | | US 2021040118 A1 | 11 February 2021 |
| | | EP 3774750 A1 | 17 February 2021 |
| | | JP 2021520342 A | 19 August 2021 |
| WO 2019191624 A1 | 03 October 2019 | CA 3093851 A1 | 03 October 2019 |
| | | US 2021052585 A1 | 25 February 2021 |
| CN 113801111 A | 17 December 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021008491 A1
- WO 2019149183 A